# EUROPÄISCHE PATENTSCHRIFT

(11) **EP 2 663 567 B1**
(45) Veröffentlichungstag und Bekanntmachung des Hinweises auf die Patenterteilung: **01.06.2016**
(21) Anmeldenummer: 11799637.1
(22) Anmeldetag: 15.12.2011
(51) Int. Cl.: H01L 51/00, C07D 487/22, C09B 57/00

(54) **VERBINDUNGEN FÜR ORGANISCHE ELEKTROLUMINESZENZVORRICHTUNGEN**
COMPOUNDS FOR ORGANIC ELECTROLUMINESCENT DEVICES
COMPOSÉS POUR DES DISPOSITIFS ÉLECTROLUMINESCENTS ORGANIQUES

(30) Priorität: 13.01.2011 EP 11000214
(43) Veröffentlichungstag der Anmeldung: 20.11.2013
(73) Patentinhaber: Merck Patent GmbH, 64293 Darmstadt (DE)
(72) Erfinder: STOESSEL, Philipp, 60487 Frankfurt am Main (DE); BUESING, Arne, 65929 Frankfurt am Main (DE); BREUNING, Esther, 64372 Ober-Ramstadt (DE); PFLUMM, Christof, 60316 Frankfurt am Main (DE); PARHAM, Amir Hossain, 65929 Frankfurt am Main (DE); EBERLE, Thomas, 76829 Landau (DE); MUJICA-FERNAUD, Teresa, 64289 Darmstadt (DE)
(86) Internationale Anmeldenummer: PCT/EP2011/006355
(87) Internationale Veröffentlichungsnummer: WO 2012/095143

(56) Entgegenhaltungen:
- WO-A1-01/49806

## Beschreibung

Die vorliegende Erfindung betrifft neue Verbindungen, welche aromatische Stickstoffheterocyclen enthalten, für die Verwendung in elektronischen Vorrichtungen, insbesondere in organischen Elektrolumineszenzvorrichtungen.

Der Aufbau organischer Elektrolumineszenzvorrichtungen (OLEDs), in denen organische Halbleiter als funktionelle Materialien eingesetzt werden, ist beispielsweise in US 4539507, US 5151629, EP 0676461 und WO 98/27136 beschrieben. Allerdings sind noch weitere Verbesserungen wünschenswert, um diese Vorrichtungen für hochwertige und langlebige Displays zu verwenden. So gibt es insbesondere bei der Lebensdauer, der Effizienz und der Betriebsspannung organischer Elektrolumineszenzvorrichtungen derzeit noch Verbesserungsbedarf. Weiterhin ist es erforderlich, dass die Verbindungen eine hohe thermische Stabilität aufweisen und sich unzersetzt sublimieren lassen.

Insbesondere bei den Ladungsinjektions- und -transportmaterialien sind noch Verbesserungen wünschenswert, da gerade auch die Eigenschaften der Ladungstransportmaterialien einen wesentlichen Einfluss auf die oben genannten Eigenschaften der organischen Elektrolumineszenzvorrichtung ausüben. Insbesondere besteht Verbesserungsbedarf bei Elektronentransportmaterialien und Lochinjektions- bzw. Lochtransportmaterialien, welche gleichzeitig zu guter Effizienz, hoher Lebensdauer und geringer Betriebsspannung führen. Gerade auch die Eigenschaften dieser Materialien sind häufig limitierend für die Lebensdauer, die Effizienz und die Betriebsspannung der organischen Elektrolumineszenzvorrichtung.

Als Lochinjektions- bzw. Lochtransportmaterialien in organischen Elektrolumineszenzvorrichtungen gemäß dem Stand der Technik werden neben Triarylaminderivaten bzw. Carbazolderivaten insbesondere auch Hexaazatriphenylenderivate, vor allem solche, die mit Cyanogruppen substituiert sind, verwendet (z. B. WO 2001/049806). Diese Verbindungen werden im Allgemeinen als separate Schicht, die an eine oder mehrere Lochtransportschichten angrenzt, oder in Mischung mit einem Lochtransportmaterial verwendet. Bei Verwendung dieser Verbindungen besteht noch Verbesserungsbedarf in Bezug auf die Lebensdauer, die Effizienz und die Betriebsspannung. Weiterhin wäre es wünschenswert, für die Verwendung in Kombination mit Triplettemittern Materialien zur Verfügung zu haben, die ein höheres Triplettniveau aufweisen.

AlQ₃ wird bereits seit langem als Elektronentransportmaterial verwendet (z. B. US 4,539,507), hat allerdings mehrere Nachteile: Es lässt sich nicht rückstandsfrei aufdampfen, da es sich bei der Sublimationstemperatur teilweise zersetzt, was insbesondere für Produktionsanlagen ein großes Problem darstellt. Dies hat zur Folge, dass die Aufdampfquellen immer wieder gereinigt oder gewechselt werden müssen. Des Weiteren gelangen Zersetzungsprodukte von AlQ₃ in die OLED, die dort zu einer verringerten Lebensdauer und reduzierten Quanten- und Leistungseffizienz beitragen. AlQ₃ hat außerdem eine niedrige Elektronenbeweglichkeit, was zu höheren Spannungen und damit zu einer niedrigeren Leistungseffizienz führt. Um Kurzschlüsse im Display zu vermeiden, würde man gern die Schichtdicke erhöhen; dies ist mit AlQ₃ wegen der geringen Ladungsträgerbeweglichkeit und der daraus resultierenden Spannungserhöhung nicht möglich. Die Ladungsträgerbeweglichkeit anderer Elektronenleiter (US 4,539,507) ist ebenfalls zu gering, um dickere Schichten damit aufzubauen, wobei die Lebensdauer der OLED noch schlechter ist als bei Verwendung von AlQ₃. Als ungünstig erweist sich auch die Eigenfarbe (im Feststoff gelb) von AIQ₃, die gerade bei blauen OLEDs durch Reabsorption und schwache Reemission zu Farbverschiebungen führen kann. Hier sind blaue OLEDs nur mit starken Effizienz- bzw. Farborteinbußen darstellbar.

Es besteht also weiterhin Bedarf an Elektronentransportmaterialien und Lochinjektions- bzw. Lochtransportmaterialien, die in organischen Elektrolumineszenzvorrichtungen zu guten Effizienzen und gleichzeitig zu hohen Lebensdauern führen. Es wurde nun überraschend gefunden, dass organische Elektrolumineszenzvorrichtungen, die bestimmte - im Folgenden aufgeführte - Stickstoffheteroaromaten als Elektronentransportmaterialien oder als Lochinjektions- bzw. Lochtransportmaterialien enthalten, sehr gute Eigenschaften aufweisen, insbesondere in Bezug auf Effizienz und Lebensdauer und insbesondere auch in Kombination mit Triplettemittern. Insbesondere werden mit diesen Materialien bessere Ergebnisse erhalten als mit den Hexaazatriphenylenderivaten gemäß dem Stand der Technik.

Gegenstand der Erfindung ist eine Verbindung der folgenden Formeln (2) bis (14), 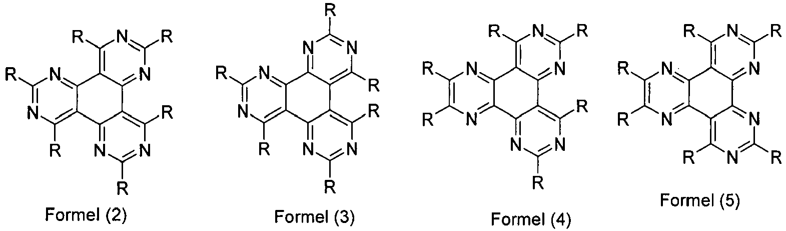 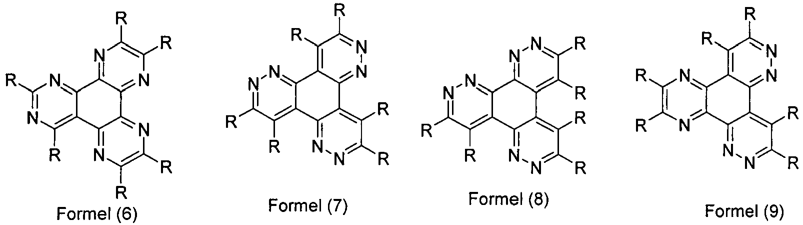 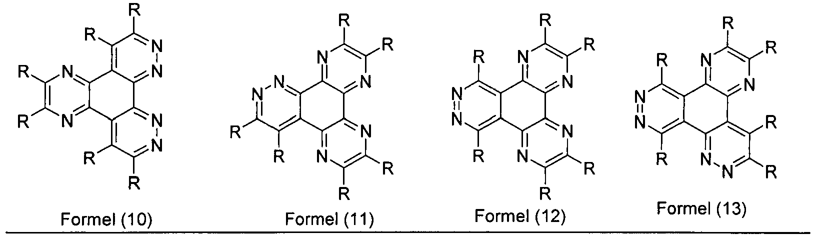 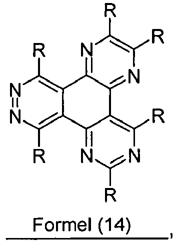 wobei für die verwendeten Symbole gilt:
- R: ist bei jedem Auftreten gleich oder verschieden H, F, C(=O)Ar, P(=O)(Ar)₂, CN, eine geradkettige Alkylgruppe mit 1 bis 10 C-Atomen oder eine verzweigte oder cyclische Alkylgruppe mit 3 bis 10 C-Atomen. die jeweils mit einem oder mehreren Resten R¹ substituiert sein kann, wobei ein oder mehrere H-Atome durch F oder CN ersetzt sein können. oder ein aromatisches oder heteroaromatisches Ringsystem mit 5 bis 40 aromatischen Ringatomen, das jeweils durch einen oder mehrere Reste R¹ substituiert sein kann
- R¹: ist bei jedem Auftreten gleich oder verschieden H, D, F, Cl, Br, I, CHO, N(R²)₂, N(Ar)₂, C(=O)Ar, P(=O)(Ar)₂, S(=O)Ar, S(=O)₂Ar, CR²=CR²Ar, CN, NO₂, Si(R²)₃, B(OR²)₂, B(R²)₂, B(N(R²)₂)₂, OSO₂R², eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R² substituiert sein kann, wobei eine oder mehrere nicht benachbarte CH₂-Gruppen durch R²C=CR², C≡C, Si(R²)₂, Ge(R²)₂, Sn(R²)₂, C=O, C=S, C=Se, C=NR², P(=O)(R²), SO, SO₂, NR², O, S oder CONR² ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ring-atomen, die durch einen oder mehrere Reste R² substituiert sein kann, oder eine Kombination dieser Systeme;
- Ar: ist bei jedem Auftreten gleich oder verschieden ein aromatisches oder heteroaromatisches Ringsystem mit 5 bis 30 aromatischen Ringatomen, das mit einem oder mehreren nicht-aromatischen Resten R¹ substituiert sein kann; dabei können auch zwei Reste Ar, welche an dasselbe Stickstoff- oder Phosphoratom binden, durch eine Einfachbindung oder eine Brücke, ausgewählt aus B(R²), C(R²)₂, Si(R²)₂, C=O, C=NR², C=C(R²)₂, O, S, S=O, SO₂, N(R²), P(R²) und P(=O)R², miteinander verknüpft sein;
- R²: ist bei jedem Auftreten gleich oder verschieden H, D oder ein aliphatischer, aromatischer und/oder heteroaromatischer Kohlenwasserstoffrest mit 1 bis 20 C-Atomen, in dem auch H-Atome durch F ersetzt sein können; dabei können zwei oder mehrere benachbarte Substituenten R² auch miteinander ein mono- oder polycyclisches, aliphatisches oder aromatisches Ringsystem bilden.

Eine Arylgruppe im Sinne dieser Erfindung enthält 6 bis 60 C-Atome; eine Heteroarylgruppe im Sinne dieser Erfindung enthält 2 bis 60 C-Atome und mindestens 1 Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe, beispielsweise Naphthalin, Anthracen, Pyren, Chinolin, Isochinolin, etc., verstanden.

Ein aromatisches Ringsystem im Sinne dieser Erfindung enthält 6 bis 60 C-Atome im Ringsystem. Ein heteroaromatisches Ringsystem im Sinne dieser Erfindung enthält 2 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine kurze, nicht-aromatische Einheit, wie z. B. ein C-, N-oder O-Atom, unterbrochen sein können. So sollen beispielsweise auch Systeme wie 9,9'-Spirobifluoren, 9,9-Diarylfluoren, Triarylamin, Diarylether, Stilben, Benzophenon, etc. als aromatische Ringsysteme im Sinne dieser Erfindung verstanden werden. Ebenso werden unter einem aromatischen bzw. heteroaromatischen Ringsystem Systeme verstanden, in denen mehrere Aryl- bzw. Heteroarylgruppen durch Einfachbindungen miteinander verknüpft sind, beispielsweise Biphenyl, Terphenyl oder Bipyridin.

Im Rahmen der vorliegenden Erfindung werden unter einer C₁- bis C₄₀-Alkylgruppe, in der auch einzelne H-Atome oder CH₂-Gruppen durch die oben genannten Gruppen substituiert sein können, besonders bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, n-Hexyl, Cyclohexyl, n-Heptyl, Cycloheptyl, n-Octyl, Cyclooctyl, 2-Ethylhexyl, Trifluormethyl, Pentafluorethyl und 2,2,2-Trifluorethyl verstanden. Unter einer Alkenylgruppe im Sinne dieser Erfindung werden insbesondere Ethenyl, Propenyl, Butenyl, Pentenyl, Cyclopentenyl, Hexenyl, Cyclohexenyl, Heptenyl, Cycloheptenyl, Octenyl und Cyclooctenyl verstanden. Unter einer Alkinylgruppe im Sinne dieser Erfindung werden insbesondere Ethinyl, Propinyl, Butinyl, Pentinyl, Hexinyl, Heptinyl oder Octinyl verstanden. Unter einer C₁- bis C₄₀-Alkoxygruppe werden besonders bevorzugt Methoxy, Trifluormethoxy, Ethoxy, n-Propoxy, i-Propoxy, n-Butoxy, i-Butoxy, s-Butoxy, t-Butoxy oder 2-Methylbutoxy verstanden. Unter einem aromatischen oder heteroaromatischen Ringsystem mit 5 - 60 aromatischen Ringatomen, welches noch jeweils mit den oben genannten Resten R substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Phenanthren, Benzanthracen, Benzphenanthren, Pyren, Chrysen, Perylen, Fluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

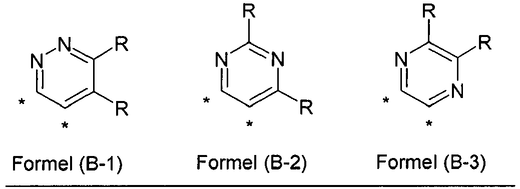

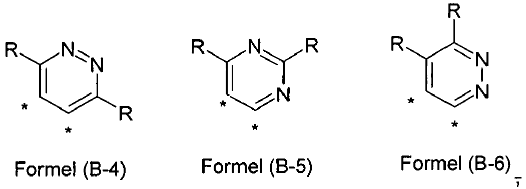

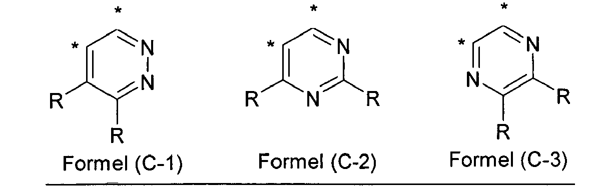

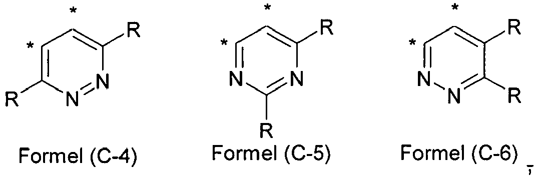

Die anspruchsgemäßen Verbindungen entsprechen den folgenden Formeln (2) bis (14), 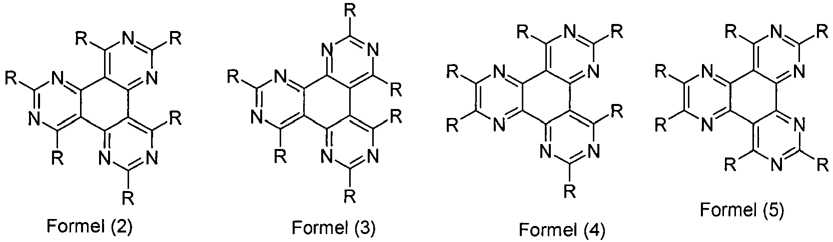 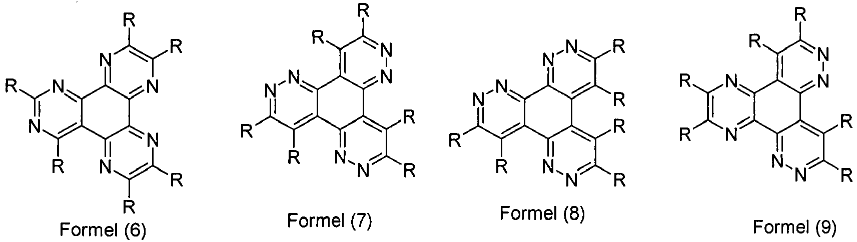 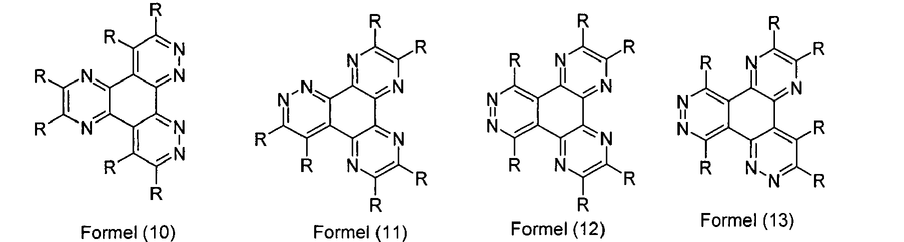 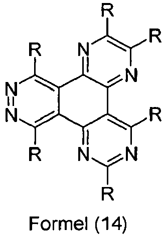 wobei die verwendeten Symbole die oben genannten Bedeutungen aufweisen.

Bevorzugt sind Verbindungen gemäß einer der Formeln (2) bis (14), in denen der heteroaromatische Grundkörper, also der Heteroaromat ohne die Reste R, C₃ₕ- oder C₂ᵥ-Symmetrie aufweist. Von den oben genannten Verbindungen weisen die Verbindungen der Formeln (2) und (7) C₃ₕ-Symmetrie auf, und die Verbindungen der Formeln (5), (10) und (12) weisen C₂ᵥ-Symmetrie auf. Diese Verbindungen sind somit bevorzugt. Ganz besonders bevorzugt sind Verbindungen mit C₃ₕ-Symmetrie, also Verbindungen der Formeln (2) und (7).

R steht in den Verbindungen der Formeln (2) bis (14) und in den Teilstrukturen der Formeln (A-1) bis (A-3), (B-1) bis (B-6) und (C-1) bis (C-6) gleich oder verschieden bei jedem Auftreten für H, F, C(=O)Ar, P(=O)(Ar)₂, CN, eine geradkettige Alkylgruppe mit 1 bis 10 C-Atomen oder eine verzweigte oder cyclische Alkylgruppe mit 3 bis 10 C-Atomen, die jeweils mit einem oder mehreren Resten R¹ substituiert sein kann, wobei ein oder mehrere H-Atome durch F oder CN ersetzt sein können, oder ein aromatisches oder heteroaromatisches Ringsystem mit 5 bis 40 aromatischen Ringatomen, das jeweils durch einen oder mehrere Reste R¹ substituiert sein kann. In einer besonders bevorzugten Ausführungsform der Erfindung steht R in den Verbindungen der Formeln (2) bis (14) gleich oder verschieden bei jedem Auftreten für H, F, CN, CF₃, oder ein aromatisches oder heteroaromatisches Ringsystem mit 5 bis 30 aromatischen Ringatomen, welches jeweils durch einen oder mehrere Reste R¹ substituiert sein kann, bevorzugt aber unsubstituiert ist. Wenn R für ein aromatisches oder heteroaromatisches Ringsystem steht, ist R bevorzugt ausgewählt aus der Gruppe bestehend aus Phenyl, 2-, 3- oder 4-Pyridyl, Pyrazinyl, 2-, 4- oder 5-Pyrimidinyl, 3- oder 4-Pyridazinyl, Triazinyl, ortho-, meta- oder para-Biphenyl, ortho-, meta- oder para-Terphenyl, Quaterphenyl, 2-Fluorenyl, 2-Spirobifluorenyl, 1-Naphthyl, 2-Naphthyl, Anthracenyl, Phenylanthracenyl, 1- oder 2-Naphthylanthracenyl, Binaphthyl, Pyrenyl, Fluoranthenyl, 2-, 3-, 4-, 5-, 6- oder 7-Benzanthracenyl, N-Imidazolyl, N-Benzimidazolyl, Phenyl-N-benzimidazolyl, N-Phenylbenzimidazolyl, Phenyl-N-phenylbenzimidazolyl oder Kombinationen dieser Gruppen, welche jeweils durch einen oder mehrere Reste R¹ substituiert sein können. In einer ganz besonders bevorzugten Ausführungsform der Erfindung ist R gleich CN.

In einer bevorzugten Ausführungsform der Erfindung sind alle Reste R gleich gewählt. Diese Bevorzugung begründet sich in der besseren synthetischen Zugänglichkeit der Verbindungen.

In einer weiteren bevorzugten Ausführungsform der Erfindung sind mindestens zwei Reste R unterschiedlich voneinander, was zu Verbindungen mit verringerter Symmetrie führt. Die Verringerung der Symmetrie kann zu Vorteilen bezüglich einer verringerten Kristallinität der Verbindungen führen. Weiterhin haben unsymmetrische Verbindungen häufig den Vorteil, dass sie eine geringere Aufdampftemperatur aufweisen.

Beispiele für bevorzugte erfindungsgemäße Verbindungen sind die im Folgenden abgebildeten Strukturen.

| | | |
|---|---|---|
| 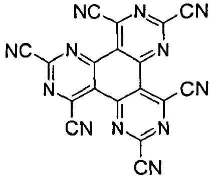 | 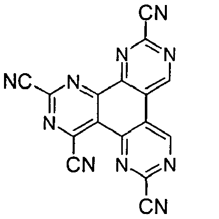 | 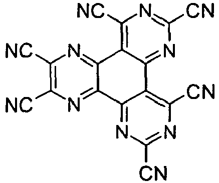 |
| (1) | (2) | (3) |
| 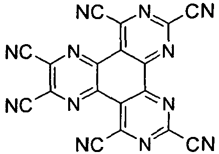 | 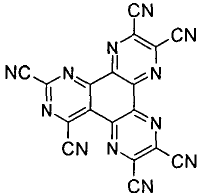 | 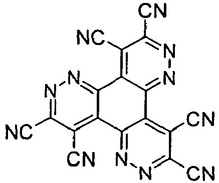 |
| (3) | (4) | (6) |
| 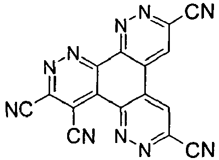 | 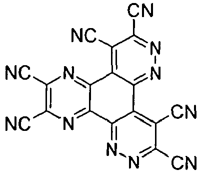 | 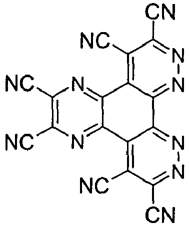 |
| (7) | (8) | (9) |
| 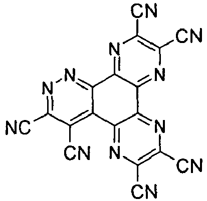 | 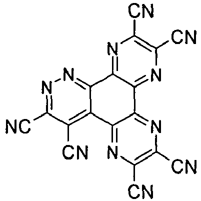 | 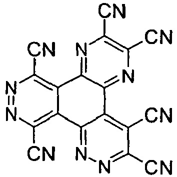 |
| (10) | (11) | (12) |
| 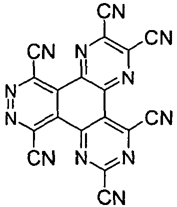 | 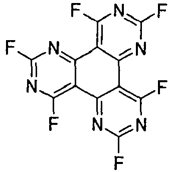 | 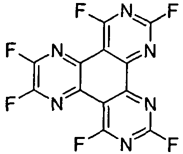 |
| (13) | (14) | (15) |
| 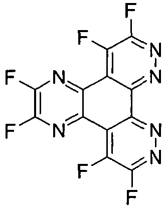 | 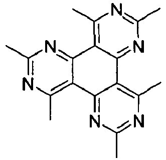 | 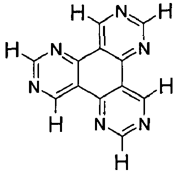 |
| (16) | (17) | (18) |
| 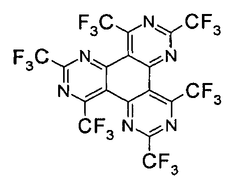 | 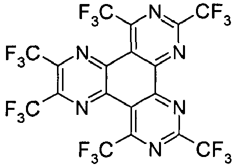 | |
| (19) | (20) | |
| 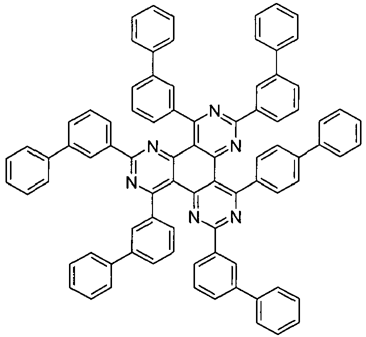 | 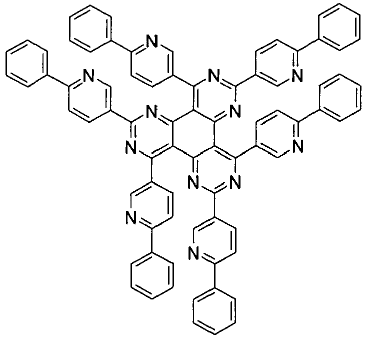 | |
| (22) | (23) | |
| 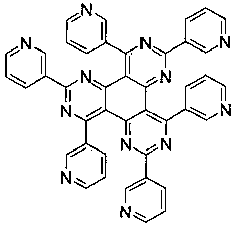 | 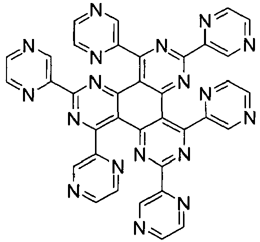 | 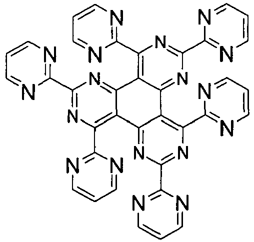 |
| (24) | (25) | (26) |
| 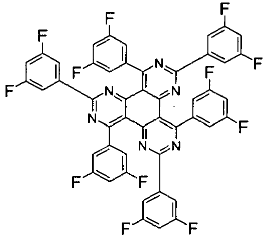 | 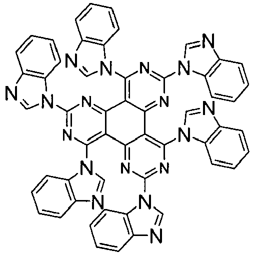 | |
| (27) | (28) | |
| 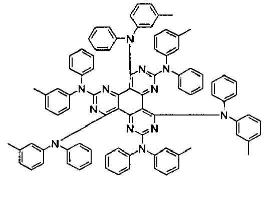 | 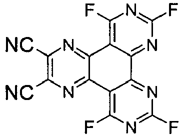 | 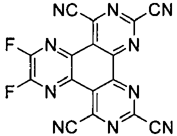 |
| (30) | (31) | (32) |
| | 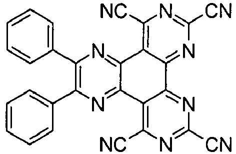 | 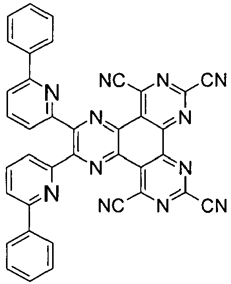 |
| | (34) | (35) |
| 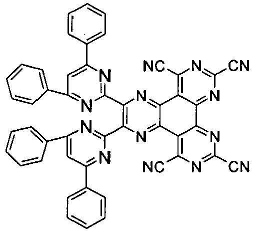 | 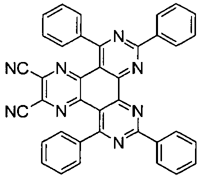 | |
| (36) | (37) | |
| 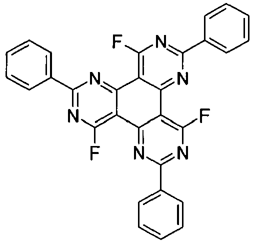 | | 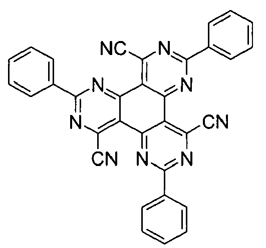 |
| (43) | | (44) |

Die Synthese der erfindungsgemäßen Verbindungen kann nach dem Fachmann der organischen Synthese allgemein bekannten Verfahrensschritten erfolgen, wie in Schema 1 für Verbindungen der Formel (2) und in Schema 2 für Verbindungen der Formel (4) exemplarisch dargestellt.

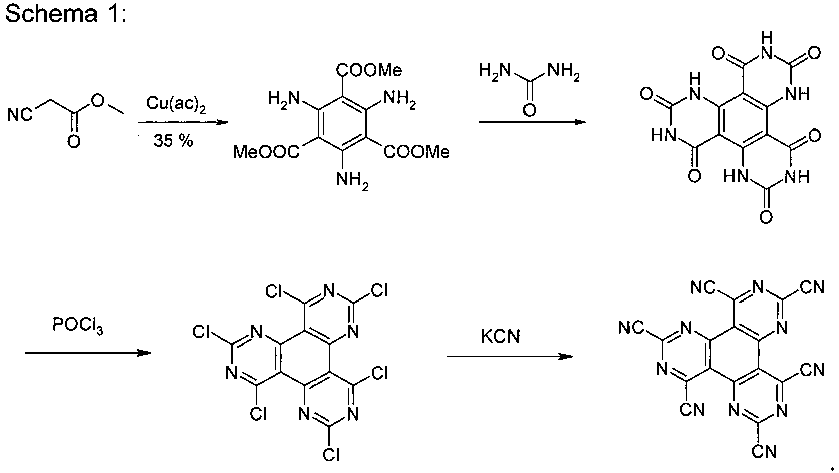

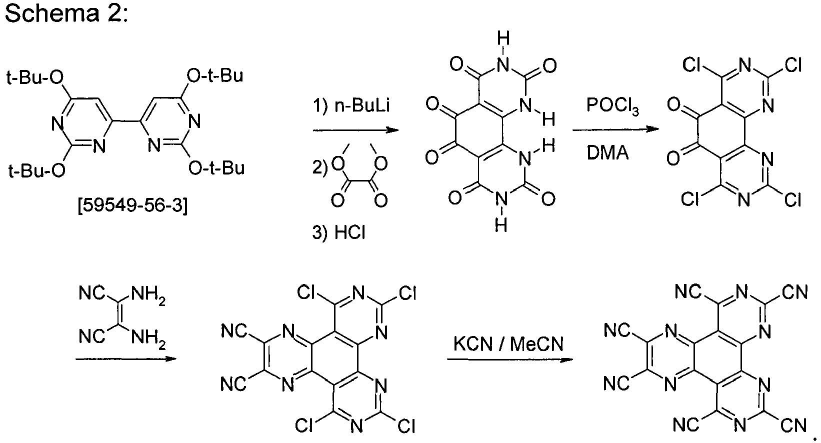

Die Chlorsubstituenten können durch nukleophile aromatische Substitution auch gegen andere Nukleophile, beispielsweise F, ausgetauscht werden, insbesondere unter Aktivierung durch eine Brönsted- oder LewisSäure. Auch die Einführung anderer Gruppen, beispielsweise substituierter Amino-, Alkoxy- oder Thioalkoxygruppen ist so möglich (Schema 3). Verbindungen mit R= Alkoxy oder Thioalkoxy sind nicht vom Umfang der Ansprüche umfasst.

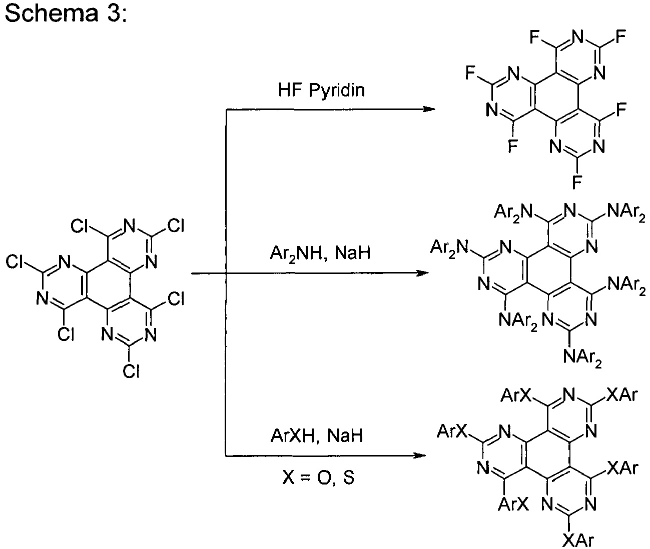

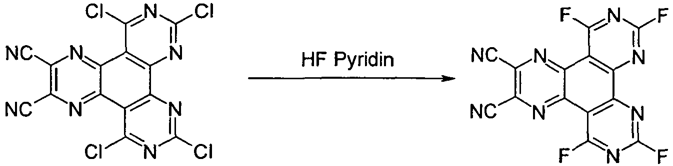

Die Einführung aromatischer oder heteroaromatischer Substituenten ist durch Reaktion der chlorierten Verbindung mit metallorganischen Derivaten von aromatischen oder heteroaromatischen Verbindungen möglich, insbesondere mit Organolithiumverbindungen oder Grignardverbindungen. Weiterhin sind palladiumkatalysierte Kupplungsreaktionen, insbesondere mit Boronsäurederivaten (Suzuki-Kupplung) oder Organozinkverbindungen (Negishi-Kupplung) zur Einführung aromatischer Substituenten möglich (Schema 4).

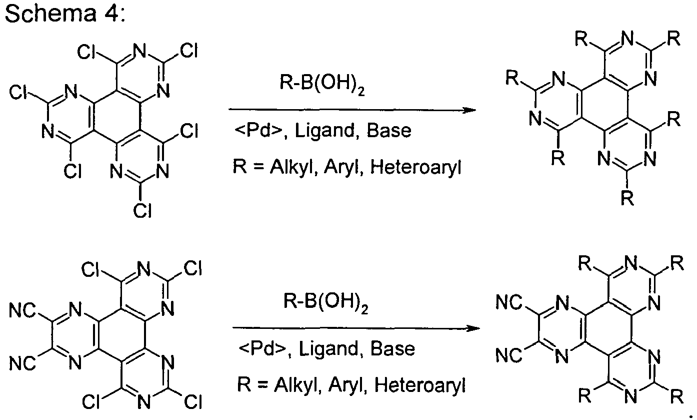

Diarylaminogruppen können auch durch palladiumkatalysierte Hartwig-Buchwald-Kupplung, Alkine über Sonogashira-Kupplung, Alkene über Heck-Kupplung eingeführt werden.

Die Halogenfunktion kann auch durch Transmetallierung mit Organolithiumverbindungen bzw. Grignardverbindungen in eine elektrophile Gruppe überführt werden, die dann mit einer Vielzahl von Elektrophilen, wie z. B. Aryl-Bor-Halogeniden, Aldehyden, Ketonen, Nitrilen, Estern, Halogenestern, Kohlendioxid, Arylphosphinhalogeniden, Halogensulfinsäuren, Halogenarylsulfonsäuren, etc., gekuppelt werden.

Ortho-Chinoide Zwischenverbindungen können mit einer Vielzahl von vicinalen Diaminen gekuppelt werden, wie dies in Schema 5 am Beispiel der Zwischenverbindung 1,3,6,8-Tetrachlor-2,4,5,7-tetraaza-phenanthren-9,10-dion gezeigt ist.

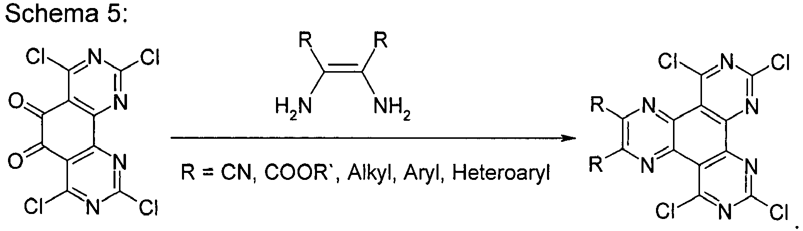

Eine Kondensation dieser Zwischenverbindungen mit 1,2-Arylenen bzw. 1,2-Heteroarylen führt zu erfindungsgemäßen Verbindungen mit erweitertem π-System (Schema 6). Hinweis: die in Schema 6 gezeigten Verbindungen sind nicht vom Umfang der Ansprüche umfasst.

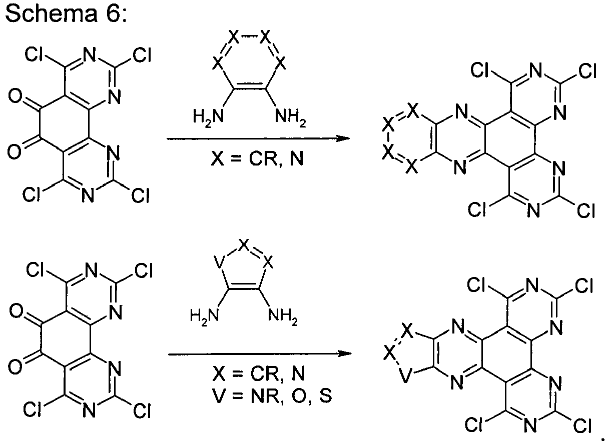

Die oben genannten Reaktionen sind dem Fachmann der organischen Chemie generell bekannt und können von ihm ohne erfinderisches Zutun auf die erfindungsgemäßen Verbindungen angewandt werden.

Ein weiterer Gegenstand der vorliegenden Erfindung ist ein Verfahren zur Herstellung der erfindungsgemäßen Verbindungen, charakterisiert durch die folgenden Reaktionsschritte:
a) Synthese des entsprechenden halogenierten Grundkörpers; und
b) Umsetzung des Halogens zum gewünschten Substituenten, insbesondere durch Umhalogeniertung, Cyanierung, Alkoxylierung, Aminierung, Arylierung und/oder Heteroarylierung.

Die oben beschriebenen erfindungsgemäßen Verbindungen, insbesondere Verbindungen, in welchen mindestens eine Gruppe R für eine reaktive Abgangsgruppe, wie Brom, Iod, Triflat, Tosylat, Boronsäure oder Boronsäureester, oder eine polymerisierbare Gruppe, wie Styrol, Alkenyl oder Acrylat, steht, können auch als Monomere zur Erzeugung entsprechender Oligomere, Polymere oder als Kern von Dendrimeren Verwendung finden. Die Oligomerisation bzw. Polymerisation erfolgt dabei bevorzugt über die Halogenfunktionalität bzw. die Boronsäurefunktionalität. Unter einem Oligomer im Sinne dieser Erfindung wird eine Verbindung verstanden, welche etwa 2 bis 9 Wiederholeinheiten aufweist. Ein Polymer im Sinne dieser Erfindung weist etwa 10 oder mehr Wiederholeinheiten auf.

Ein weiterer Gegenstand der Erfindung sind daher Oligomere, Polymere oder Dendrimere, welche eine oder mehrere erfindungsgemäße Verbindungen enthalten, wobei ein oder mehrere Reste R Bindungen der Verbindung zum Polymer, Oligomer oder Dendrimer darstellen. Die Polymere, Oligomere oder Dendrimere können konjugiert, teilkonjugiert oder nicht-konjugiert sein. Die Oligomere oder Polymere können linear oder verzweigt sein. In den linear verknüpften Strukturen können die erfindungsgemäßen Einheiten direkt miteinander verknüpft sein oder sie können über eine bivalente Gruppe, wie z. B. über eine substituierte oder unsubstituierte Alkylengruppe, über ein Heteroatom oder über eine bivalente aromatische oder heteroaromatische Gruppe, miteinander verknüpft sein. In verzweigten Strukturen können beispielsweise drei oder mehrere erfindungsgemäße Einheiten über eine trivalente oder höhervalente Gruppe, beispielsweise über eine trivalente oder höhervalente aromatische oder heteroaromatische Gruppe, zu einem verzweigten Oligomer oder Polymer verknüpft sein.

Zur Herstellung der Oligomere oder Polymere werden die entsprechenden Monomere homopolymerisiert oder mit weiteren Monomeren copolymerisiert. Geeignete und bevorzugte Comonomere sind gewählt aus Fluorenen (z. B. gemäß EP 842208 oder WO 00/22026), Spirobifluorenen (z. B. gemäß EP 707020, EP 894107 oder WO 2006/061181), Para-phenylenen (z. B. gemäß WO 92/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689), cis- und trans-Indeno-fluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine (z. B. gemäß WO 2007/068325) oder phosphoreszierende Metallkomplexe (z. B. gemäß WO 2006/003000), und/oder Ladungstransporteinheiten. Dabei eignen sich die erfindungsgemäßen Wiederholeineiten insbesondere als Ladungstransporteinheiten für Elektronen.

Die erfindungsgemäßen Verbindungen eignen sich für den Einsatz in elektronischen Vorrichtungen, insbesondere in organischen Elektrolumineszenzvorrichtungen.

Ein weiterer Gegenstand der vorliegenden Erfindung ist daher die Verwendung einer erfindungsgemäßen Verbindung in einer elektronischen Vorrichtung, insbesondere in einer organischen Elektrolumineszenzvorrichtung.

Ein weiterer Gegenstand der vorliegenden Erfindung ist eine elektronische Vorrichtung enthaltend mindestens eine erfindungsgemäße Verbindung. Dabei sind für die elektronische Vorrichtung dieselben Verbindungen bevorzugt, wie sie oben beim Stoffschutz ausgeführt sind.

Unter einer elektronischen Vorrichtung im Sinne der vorliegenden Erfindung wird eine Vorrichtung verstanden, welche Anode und Kathode und mindestens eine zwischen Anode und Kathode angeordnete Schicht enthält, wobei diese Schicht mindestens eine organische oder metallorganische Verbindung enthält. Es ist jedoch nicht notwendig, dass die Vorrichtung nur organische Schichten enthält. So können auch eine oder mehrere Schichten vorhanden sein, welche anorganische Materialien enthalten oder vollständig aus anorganischen Materialien bestehen. Ebenso können Anode und Kathode aus rein anorganischen Materialien bestehen oder solche enthalten.

Die elektronische Vorrichtung ist insbesondere ausgewählt aus der Gruppe bestehend aus organischen Elektrolumineszenzvorrichtungen (OLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), farbstoffsensibilisierten Solarzellen (DSSCs), organischen optischen Detektoren, organischen Photorezeptoren, organischen Feld-Quench-Devices (O-FQDs), lichtemittierenden elektrochemischen Zellen (LECs), organischen Laserdioden (O-Laser) und "organic plasmon emitting device" (D. M. Koller et al., Nature Photonics 2008, 1-4), insbesondere aber organischen Elektrolumineszenzvorrichtungen, organische Solarzellen und organische Feld-Effekt-Transistoren.

Die organische Elektrolumineszenzvorrichtung enthält Anode, Kathode und mindestens eine emittierende Schicht, wobei mindestens eine organische Schicht, die die emittierende Schicht oder eine andere Schicht sein kann, mindestens eine erfindungsgemäße Verbindung oder ein entsprechendes Oligomer, Polymer oder Dendrimer enthält.

Außer Kathode, Anode und der emittierenden Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Exzitonenblockierschichten, Ladungserzeugungsschichten (Charge-Generation Layers) und/oder organischen oder anorganischen p/n-Übergängen. Weiterhin können die Schichten, insbesondere die Ladungstransportschichten, auch dotiert sein. Die Dotierung der Schichten kann für einen verbesserten Ladungstransport vorteilhaft sein. Es sei aber darauf hingewiesen, dass nicht notwendigerweise jede dieser Schichten vorhanden sein muss und die Wahl der Schichten immer von den verwendeten Verbindungen abhängt und insbesondere auch von der Tatsache, ob es sich um eine fluoreszierende oder phosphoreszierende Elektrolumineszenzvorrichtung handelt.

In einer Ausführungsform der Erfindung enthält die organische Elektrolumineszenzvorrichtung mehrere emittierende Schichten, wobei mindestens eine organische Schicht, die eine emittierende Schicht oder eine andere Schicht sein kann, mindestens eine erfindungsgemäße Verbindung enthält. Besonders bevorzugt weisen diese Emissionsschichten insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues und gelbes, orange oder rotes Licht emittieren. Insbesondere bevorzugt sind Dreischichtsysteme, also Systeme mit drei emittierenden Schichten, wobei die drei Schichten blaue, grüne und orange oder rote Emission zeigen (für den prinzipiellen Aufbau siehe z. B. WO 2005/011013). Ebenso eignen sich für weiße Emission Emitter, welche breitbandige Emissionsbanden aufweisen und dadurch weiße Emission zeigen. Ebenso lässt sich eine weiß emittierende OLED als Tandem-OLED realisieren.

In einer bevorzugten Ausführungsform der Erfindung werden die erfindungsgemäßen Verbindungen in einer Lochinjektions- bzw. Lochtransportschicht verwendet. Dies gilt insbesondere, wenn mindestens ein Substituent R, bevorzugt mindestens zwei Substituenten R, besonders bevorzugt mindestens drei Substituenten R, ganz besonders bevorzugt alle Substituenten R für eine elektronenarme Gruppe stehen. Im Gegensatz zu Triarylaminderivaten, die üblicherweise in der Lochinjektions- bzw. Lochtransportschicht verwendet werden und bei denen der Lochtransport über das HOMO ("highest occupied molecular orbital", höchstes besetztes Molekülorbital) der entsprechenden Verbindung erfolgt, erfolgt bei den erfindungsgemäßen Verbindungen der Lochtransport nicht über das HOMO, sondern über das LUMO ("lowest unoccupied molecular orbital", niedrigstes unbesetztes Molekülorbital). Besonders bevorzugte Substituenten R sind dann ausgewählt aus der Gruppe bestehend aus CN, F, CF₃ und substituierten oder unsubstituierten elektronenarmen Heterocyclen. Dabei sind die elektronenarmen Heterocyclen bevorzugt ausgewählt aus Pyridin, Pyrazin, Pyrimidin, Pyridazin, Triazin, Pyrrol, Imidazol, Triazol, Benzimidazol, Chinolin, Isochinolin, Chinoxalin, Thiadiazol, Thiazol, Oxadiazol oder heteroaromatischen Ringsystemen, welche diese Heteroarylgruppen enthalten; dabei können diese jeweils durch einen oder mehrere Reste R¹ substituiert sein. Da das LUMO dieser Verbindungen gleich tief oder sogar noch tiefer liegt im Vergleich zu den Hexaazatriphenylenderivaten, die gemäß dem Stand der Technik als Lochinjektionsmaterialien verwendet werden, eignen sich die erfindungsgemäßen Verbindungen genauso gut oder besser als die Materialien gemäß dem Stand der Technik als Lochinjektions- bzw. Lochtransportmaterialien. Viele der erfindungsgemäßen Verbindungen weisen zudem ein höheres Triplettniveau auf im Vergleich zu den Verbindungen gemäß dem Stand der Technik. Dabei soll unter einem Lochinjektionsmaterial im Sinne dieser Erfindung eine Verbindung verstanden werden, welche in einer Lochinjektionsschicht eingesetzt wird. Eine Lochinjektionsschicht im Sinne dieser Erfindung ist eine Schicht, welche direkt an die Anode angrenzt. Üblicherweise folgt der Lochinjektionsschicht im Aufbau der orgaischen Elektrolumineszenzvorrichtung eine Lochtransportschicht, so dass die Lochinjektionsschicht zwischen der Anode und einer Lochtransportschicht liegt. Eine Lochtransportschicht im Sinne der vorliegenden Erfindung ist eine Schicht, welche zwischen einer Lochinjektionsschicht und der emittierenden Schicht liegt.

In einer bevorzugten Ausführungsform der Erfindung umfasst die erfindungsgemäße Elektrolumineszenzvorrichtung einen Aufbau, enthaltend in dieser Reihenfolge: Anode - Lochinjektionsschicht enthaltend mindestens eine erfindungsgemäße Verbindung - Lochtransportschicht, bevorzugt enthaltend mindestens ein Triarylaminderivat - emittierende Schicht - Kathode. Ebenso ist es in diesem Aufbau möglich, zwei oder mehrere Lochtransportschichten zu verwenden, die bevorzugt alle mindestens ein Triarylaminderivat enthalten. Ein weiterer bevorzugter Aufbau der Elektrolumineszenzvorrichtung enthält in dieser Reihenfolge: Anode - Lochinjektionsschicht, bevorzugt enthaltend mindestens ein Triarylaminderivat - Lochtransportschicht, enthaltend mindestens eine erfindungsgemäße Verbindung - Lochtransportschicht, bevorzugt enthaltend mindestens ein Triarylaminderivat - emittierende Schicht - Kathode. Ebenso ist in diesem Aufbau möglich, dass zwischen die Lochinjektionsschicht und die Schicht enthaltend die erfindungsgemäße Verbindung eine weitere Lochtransportschicht, bevorzugt enthaltend mindestens ein Triarylaminderivat, eingebracht ist und/oder dass statt einer Lochtransportschicht, welche bevorzugt ein Triarylaminderivat enthält, zwei oder mehrere Lochtransportschichten, welche bevorzugt jeweils mindestens ein Triarylaminderivat enthalten, zwischen der Schicht enthaltend die erfindungsgemäße Verbindung und der emittierenden Schicht verwendet werden. Außerdem können diese Vorrichtungen weiterhin eine oder mehrere der oben aufgeführten weiteren Schichten, beispielsweise Elektronentransportschichten etc., enthalten. Dabei können die Lochtransportschichten jeweils auch p-dotiert sein.

In nochmals einer weiteren Ausführungsform der Erfindung werden die erfindungsgemäßen Verbindungen als Elektronentransportmaterial bzw. als Lochblockiermaterial in einer Elektronentransportschicht bzw. einer Lochblockierschicht eingesetzt. Eine Lochblockierschicht im Sinne dieser Erfindung ist eine Schicht, die zwischen einer emittierenden Schicht und einer Elektronentransportschicht liegt und direkt an die emittierende Schicht angrenzt. Hier ist es jeweils bevorzugt, wenn die Substituenten R gleich oder verschieden bei jedem Auftreten für ein aromatisches oder heteroaromatisches Ringsystem stehen, welche bevorzugt ausgewählt sind aus den oben genannten Gruppen. Weiterhin kann es bevorzugt sein, wenn die Verbindung mit Elektronendonorverbindungen dotiert ist. Dies gilt insbesondere für die Verwendung in einer Elektronentransportschicht. Geeignete Dotanden sind Alkalimetalle oder Alkalimetallkomplexe bzw. -verbindungen, insbesondere Lithiumverbindungen, beispielsweise Lithiumchinolinat.

In nochmals einer weiteren Ausführungsform der Erfindung werden die erfindungsgemäßen Verbindungen als Ladungserzeugungsmaterial in einer Ladungserzeugungsschicht (charge generation layer) eingesetzt. Diese findet Anwendung beispielsweise in einer Tandem-OLED.

In nochmals einer weiteren Ausführungsform der Erfindung werden die erfindungsgemäßen Verbindungen als Matrixmaterial für eine emittierende Verbindung, insbesondere für eine phosphoreszierende Verbindung eingesetzt. Dies gilt insbesondere für Verbindungen, in welchen R für eine Aryl- oder Heteroarylgruppe steht. Die phosphoreszierende Verbindung ist dabei bevorzugt eine rot oder grün phosphoreszierende Verbindung.

In den oben genannten Funktionen, also insbesondere als Lochinjektions- bzw. -transportmaterial, als Elektronentransportmaterial oder als Ladungserzeugungmaterial, eignen sich die Materialien auch für andere organische elektronische Vorrichtungen, wie sie oben genannt wurden.

Als Kathode der erfindungsgemäßen elektronischen Vorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Bei mehrlagigen Strukturen können auch zusätzlich zu den genannten Metallen weitere Metalle verwendet werden, die eine relativ hohe Austrittsarbeit aufweisen, wie z. B. Ag, wobei dann in der Regel Kombinationen der Metalle, wie beispielsweise Mg/Ag, Ca/Ag oder Ba/Ag, verwendet werden. Ebenso bevorzugt sind Metalllegierungen, insbesondere Legierungen aus einem Alkalimetall oder Erdalkalimetall und Silber, besonders bevorzugt eine Legierung aus Mg und Ag. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride oder die entsprechenden Oxide oder Carbonate in Frage (z. B. LiF, Li₂O, CsF, Cs₂CO₃, BaF₂, MgO, NaF, etc.), aber auch andere Alkali- und Erdalkalimetallverbindungen, wie z. B. Lithiumchinolinat. Die Schichtdicke dieser Zwischenschicht beträgt bevorzugt zwischen 0.5 und 5 nm.

Als Anode der erfindungsgemäßen elektronischen Vorrichtung sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (O-SC) oder die Auskopplung von Licht (OLED/PLED, O-Laser) zu ermöglichen. Bevorzugte Anodenmaterialien für transparente oder teiltransparente Anoden sind leitfähige gemischte Metalloxide. Besonders bevorzugt sind Indium-Zinn-Oxid (ITO) oder Indium-Zink-Oxid (IZO). Bevorzugt sind weiterhin leitfähige, dotierte organische Materialien, insbesondere leitfähige dotierte Polymere.

Die Vorrichtung wird entsprechend (je nach Anwendung) strukturiert, kontaktiert und schließlich hermetisch versiegelt, da sich die Lebensdauer derartiger Vorrichtungen bei Anwesenheit von Wasser und/oder Luft drastisch verkürzt.

Es können generell alle weiteren Materialien, wie sie gemäß dem Stand der Technik in organischen Elektrolumineszenzvorrichtungen eingesetzt werden, auch in Kombination mit den erfindungsgemäßen Verbindungen eingesetzt werden. Die emittierende Schicht kann dabei fluoreszierende und/oder phosphoreszierende Dotanden, bevorzugt jeweils in Kombination mit einem Matrixmaterial (Hostmaterial), enthalten.

Geeignete fluoreszierende Dotanden sind ausgewählt aus der Klasse der Monostyrylamine, der Distyrylamine, der Tristyrylamine, der Tetrastyrylamine, der Styrylphosphine, der Styrylether und der Arylamine. Unter einem Monostyrylamin wird eine Verbindung verstanden, die eine substituierte oder unsubstituierte Styrylgruppe und mindestens ein, bevorzugt aromatisches, Amin enthält. Unter einem Distyrylamin wird eine Verbindung verstanden, die zwei substituierte oder unsubstituierte Styrylgruppen und mindestens ein, bevorzugt aromatisches, Amin enthält. Unter einem Tristyrylamin wird eine Verbindung verstanden, die drei substituierte oder unsubstituierte Styrylgruppen und mindestens ein, bevorzugt aromatisches, Amin enthält. Unter einem Tetrastyrylamin wird eine Verbindung verstanden, die vier substituierte oder unsubstituierte Styrylgruppen und mindestens ein, bevorzugt aromatisches, Amin enthält. Die Styrylgruppen sind besonders bevorzugt Stilbene, die auch noch weiter substituiert sein können. Entsprechende Phosphine und Ether sind in Analogie zu den Aminen definiert. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte Dotanden sind gewählt aus Indenofluorenaminen bzw. -diaminen, beispielsweise gemäß WO 2006/122630, Benzoindenofluorenaminen bzw. -diaminen, beispielsweise gemäß WO 2008/006449, und Dibenzoindenofluorenaminen bzw. -diaminen, beispielsweise gemäß WO 2007/140847. Beispiele für Dotanden aus der Klasse der Styrylamine sind substituierte oder unsubstituierte Tristilbenamine oder die Dotanden, die in WO 2006/000388, WO 2006/058737, WO 2006/000389, WO 2007/065549 und WO 2007/115610 beschrieben sind. Weiterhin bevorzugt sind die in WO 2010/012328 offenbarten kondensierten Kohlenwasserstoffe.

Geeignete Hostmaterialien für die fluoreszierenden Emitter sind ausgewählt aus den Klassen der Oligoarylene (z. B. 2,2',7,7'-Tetraphenylspiro-bifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052), der Benzanthracene (z. B. gemäß WO 2008/145239) oder der Benzophenanthrene (z. B. gemäß WO 2010/083869). Besonders bevorzugte Hostmaterialien sind ausgewählt aus den Klassen der Oligoarylene, enthaltend Naphthalin, Anthracen, Benzanthracen, Benzophenanthren und/oder Pyren oder Atropisomere dieser Verbindungen, der Oligoarylenvinylene, der Ketone, der Phosphinoxide und der Sulfoxide. Ganz besonders bevorzugte Hostmaterialien sind ausgewählt aus den Klassen der Oligoarylene, enthaltend Anthracen, Benzanthracen, Benzophenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind. Geeignete Hostmaterialien sind weiterhin beispielsweise Materialien, wie sie in WO 2004/018587, WO 2008/006449, US 5935721, US 2005/0181232, JP 2000/273056, EP 681019, US 2004/0247937 und US 2005/0211958 offenbart werden.

Als phosphoreszierende Verbindungen eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten. Im Sinne der vorliegenden Anmeldung werden alle lumineszierenden Metallkomplexe, die die oben genannten Metall enthalten, als phosphoreszierende Verbindungen bezeichnet.

Beispiele für geeignete phosphoreszierende Emitter können den Anmeldungen WO 00/70655, WO 2001/41512, WO 2002/02714, WO 2002/15645, EP 1191613, EP 1191612, EP 1191614, WO 2004/081017, WO 2005/033244, WO 2005/042550, WO 2005/113563, WO 2006/008069, WO 2006/061182, WO 2006/081973, WO 2009/146770, WO 2010/086089 und den nicht offen gelegten Anmeldung EP 10006208.2 und DE 102010027317.1 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Verbindungen verwenden.

Geeignete Matrixmaterialien für den phosphoreszierenden Emitter sind ausgewählt aus der Gruppe bestehend aus aromatischen Ketonen, Phosphinoxiden, Sulfoxiden und Sulfonen, z. B. gemäß WO 2004/013080, WO 2004/093207, WO 2006/005627 oder der WO 2010/006680, Triarylaminen, Carbazolderivaten, z. B. CBP (N,N-Biscarbazolylbiphenyl) oder die in WO 2005/039246, US 2005/0069729, JP 2004/288381, EP 1205527 oder WO 2008/086851 offenbarten Carbazolderivate, cis- und trans-Indolocarbazolderivaten, z. B. gemäß WO 2007/063754 oder WO 2008/056746, cis- und trans-Indenocarbazolderivaten, z. B. gemäß WO 2010/136109, Azacarbazolen, z. B. gemäß EP 1617710, EP 1617711, EP 1731584, JP 2005/347160, bipolaren Matrixmaterialien, z. B. gemäß WO 2007/137725, Silanen, z. B. gemäß WO 2005/111172, Azaborolen oder Boronestern, z. B. gemäß WO 2006/117052, Triazinderivaten, z. B. gemäß WO 2010/015306, WO 2007/063754 oder WO 2008/056746, oder Zinkkomplexen, z. B. gemäß WO 2009/062578.

Geeignete Ladungstransportmaterialien, wie sie in der Lochinjektions- bzw. Lochtransportschicht oder in der Elektronentransportschicht der erfindungsgemäßen organischen Elektrolumineszenzvorrichtung verwendet werden können, sind außer den erfindungsgemäßen Materialien beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Beispiele für bevorzugte Lochtransportmaterialien, die in einer Lochtransport- oder Lochinjektionsschicht in der erfindungsgemäßen Elektrolumineszenzvorrichtung verwendet werden können, sind Indenofluorenamine und Derivate (z. B. gemäß WO 2006/122630, WO 2006/100896 oder DE 102008024182), die in EP 1661888 offenbarten Aminderivate, Hexaazatriphenylenderivate (z. B. gemäß WO 2001/049806), Aminderivate mit kondensierten Aromaten (z. B. gemäß US 5,061,569), die in WO 95/09147 offenbarten Aminderivate, Monobenzoindenofluorenamine (z. B. gemäß WO 2008/006449) oder Dibenzoindenofluorenamine (z. B. gemäß WO 2007/140847). Weiterhin geeignete Lochtransport- und Lochinjektionsmaterialien sind Derivate der oben genannten Verbindungen, wie sie in JP 2001/226331, EP 676461, EP 650955, WO 01/049806, US 4780536, WO 98/30071, EP 891121, EP 1661888, JP 2006/253445, EP 650955, WO 2006/073054 und US 5061569 offenbart werden.

Geeignete Lochtransport- oder Lochinjektionsmaterialien sind weiterhin beispielsweise die in der folgenden Tabelle aufgeführten Materialien.

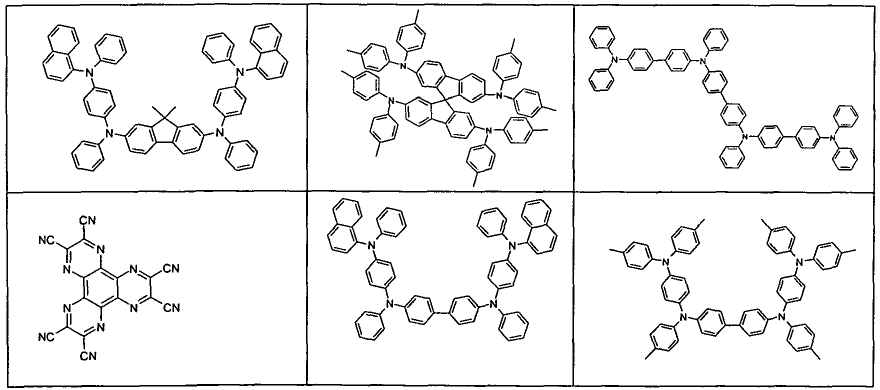

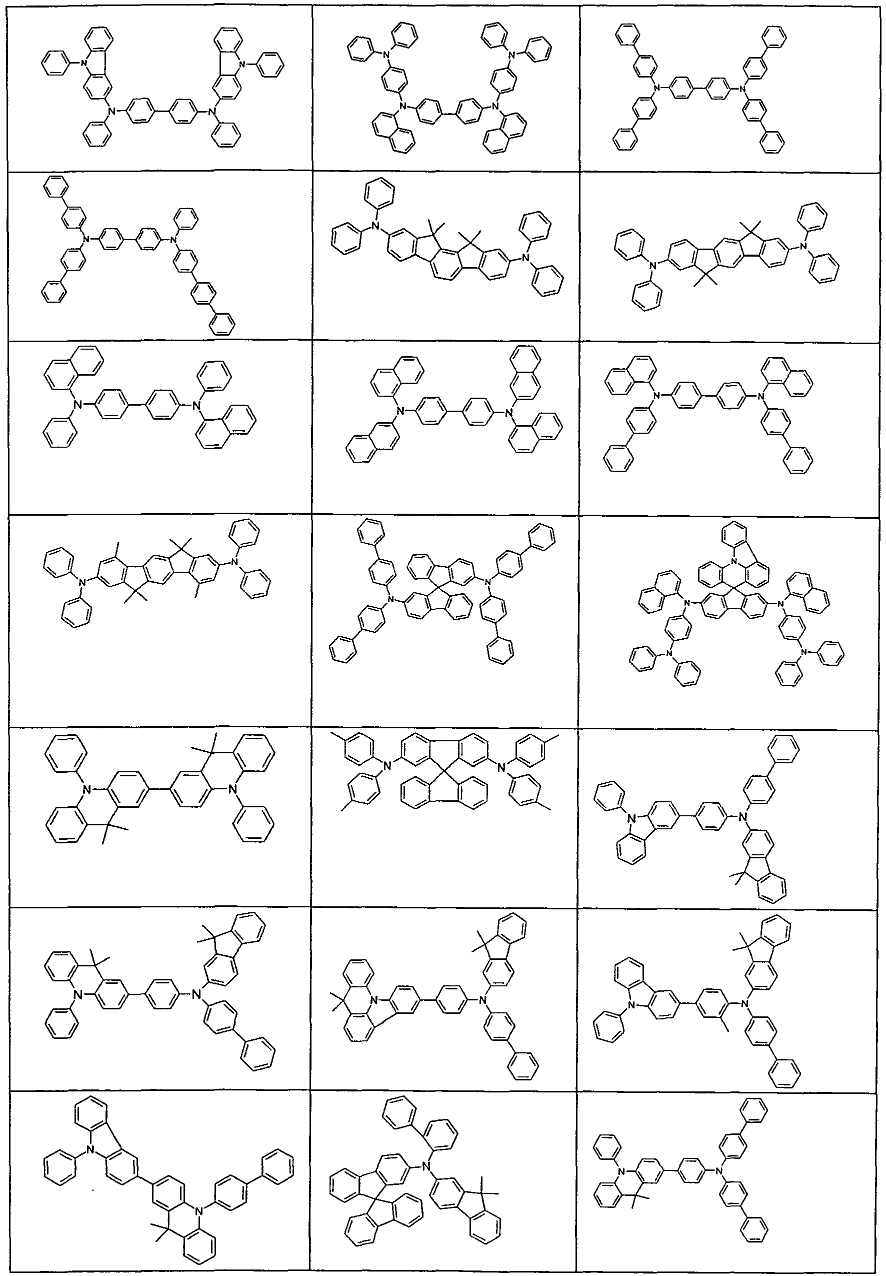

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es sei jedoch angemerkt, dass der Anfangsdruck auch noch geringer sein kann, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen nötig. Hohe Löslichkeit lässt sich durch geeignete Substitution der Verbindungen erreichen. Dabei können nicht nur Lösungen aus einzelnen Materialien aufgebracht werden, sondern auch Lösungen, die mehrere Verbindungen enthalten, beispielsweise Matrixmaterial und Dotand.

Es ist auch möglich, mehrere dieser Verfahren zu kombinieren und beispielsweise eine oder mehrere Schichten aufzudampfen und eine oder mehrere weitere Schichten aus Lösung aufzubringen.

Die oben genannten Verfahren sind ein weiterer Gegenstand der vorliegenden Erfindung.

Die erfindungsgemäßen Verbindungen weisen bei Verwendung in organischen Elektrolumineszenzvorrichtungen folgende überraschende Vorteile gegenüber dem Stand der Technik auf:
1. Die erfindungsgemäßen Verbindungen weisen eine hohe thermische Stabilität auf und lassen sich unzersetzt sublimieren.
2. Die erfindungsgemäßen Verbindungen, welche mit elektronenarmen Substituenten, insbesondere F, CN und/oder elektronenarmen Heterocyclen, substituiert sind, eignen sich sehr gut als Lochinjektionsmaterial bzw. als Lochtransportmaterial für die Verwendung in einer Lochinjektionsschicht bzw. in einer Lochtransportschicht und führen bei dieser Verwendung zu hohen Effizienzen, insbesondere zu hohen Leistungseffizienzen, und langen Lebensdauern.
3. Die erfindungsgemäßen Verbindungen, insbesondere solche, welche mit aromatischen oder heteroaromatischen Gruppen substituiert sind, eignen sich sehr gut als Elektronentransportmaterial oder als Lochblockiermaterial für die Verwendung in einer Elektronentransportschicht bzw. in einer Lochblockierschicht und führen bei dieser Verwendung zu hohen Effizienzen, insbesondere zu hohen Leistungseffizienzen, und langen Lebensdauern.
4. Die erfindungsgemäßen Verbindungen haben ein höheres Triplettniveau als Hexaazatriphenylenderivate gemäß dem Stand der Technik. Damit eignen sie sich besser als die Materialien gemäß dem Stand der Technik für die Verwendung in Kombination mit Triplettemittern, insbesondere wenn die erfindungsgemäßen Verbindungen als Triplettmatrixmaterial verwendet werden oder in einer Schicht eingesetzt werden, die direkt an eine phosphoreszierende Schicht angrenzt.

Die Erfindung wird durch die nachfolgenden Beispiele genauer beschrieben, ohne sie dadurch einschränken zu wollen. Der Fachmann kann ohne erfinderisch tätig zu werden weitere erfindungsgemäße elektronische Vorrichtungen, insbesondere organische Elektrolumineszenzvorrichtungen, herstellen.

### Beispiele:

Die nachfolgenden Synthesen werden, sofern nicht anders angegeben, unter einer Schutzgasatmosphäre in getrockneten Lösungsmitteln durchgeführt. Die Lösungsmittel und Reagenzien können von ALDRICH bzw. ABCR bezogen werden. Die Angaben in eckigen Klammern stellen jeweils die CAS-Nummer der literaturbekannten Edukte dar.

### Beispiel 1: 2,4,6,8,10,12-Hexacyano-1,3,5,7,9,11-hexaazatriphenylen

### A) 1H,3H,5H,7H,9H,11H-1,3,5,7,9,11-Hexaazatriphenylen-2,4,6,8,10,12-hexa-on

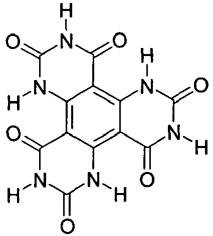

Eine Schmelze aus 148.6 g (500 mmol) 2,4,6-Triamino-1,3,5-benzoltricarbonsäure-trimethylester [139286-26-3] und 450.5 g (7.5 mol) Harnstoff werden aufgeschmolzen und so lange (ca. 4 h) auf 200 °C erhitzt, bis sich die Masse verfestigt, wobei entstehendes Methanol und Wasser abdestilliert werden. Nach Erkalten wird die Masse in 2000 ml Wasser eingetragen und unter gutem Rühren zerkleinert. Der so erhaltene sandfarbene Feststoff wird abgesaugt und erneut bei 70 °C mit 2000 ml Wasser ausgerührt. Nach Absaugen wird der Feststoff fünfmal mit je 500 ml Wasser gewaschen und dann im Vakuum getrocknet. Ausbeute: 125.8 g (381 mmol), 76 %.

### B) 2,4,6,8,10,12-Hexachlor-1,3,5,7,9,11-hexaazatriphenylen

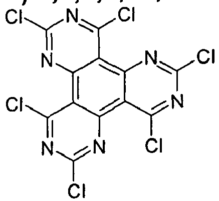

Eine Suspension von 33.0 g (100 mmol) 1H,3H,5H,7H,9H,11H-1,3,5,7,9,11-Hexaazatriphenylen-2,4,6,8,10,12-hexa-on in 185 ml Phosphorylchlorid wird tropfenweise mit 85 ml N,N-Dimethylanilin versetzt. Die Reaktionsmischung wird 20 h unter Rückfluss erhitzt, dann werden bei 50 °C alle flüchtigen Komponenten im Vakuum abgezogen. Der braune Rückstand wird mit einem Gemisch aus 500 g Eis und 500 ml Wasser versetzt und 1 h gerührt. Man saugt vom braunen Feststoff ab, wäscht diesen dreimal mit je 100 ml Wasser und trocknet dann im Vakuum. Das Rohprodukt wird aus Acetonitril umkristallisiert. Ausbeute: 26.9 g (61 mmol), 61 %.

### C) 2,4,6,8,10,12-Hexacyano-1,3,5,7,9,11-hexaaza-triphenylen

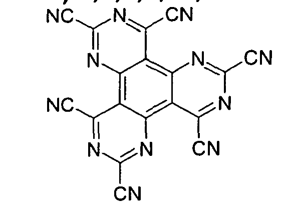

Eine Suspension von 22.0 g (50 mmol)) 2,4,6,8,10,12-Hexachlor-1,3,5,7,9,11-hexaazatriphenylen und 26.0 g (400 mmol) Kaliumcyanid in 300 ml wasserfreiem Acetonitril wird 100 h bei Raumtemperatur gerührt. Man gießt die Reaktionsmischung in ein gut gerührtes Gemisch aus 1000 g Eis und 500 ml Wasser, saugt vom entstandenen Niederschlag ab, wäscht diesen dreimal mit 200 ml Wasser und trocknet im Vakuum. Das Rohprodukt wird in 3000 ml Acetonitril in der Wärme gelöst und über Alox, sauer, Aktivitätsstufe 1 (400 g) eluiert. Nach Entfernen des Acetonitrils im Vakuum wird der Rückstand zweimal fraktioniert sublimiert (p ca. 10⁻⁵ mbar, T = 380 °C). Ausbeute: 12.3 g (32 mmol), 64 %; Reinheit: 99.9 % n. HPLC.

### Beispiel 2: 2,5,7,9,10,12-Hexacyano-1,3,4,6,8,11-hexaazatriphenylen

### A) 4H,5H,-2,4,5,7-Tetraazaphenanthren-1,3,6,8,9,10-hexa-on

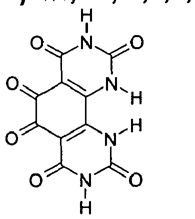

Eine auf -78 °C gekühlte Lösung von 44.7 g (100 mmol) 2,2',6,6'-Tetrakistert-butoxy-4,4'-bipyrimidin [59549-56-3] in 500 ml Diethylether wird tropfenweise mit 84.0 ml (210 mmol) n-Butyllithium (2.5 molar in n-Hexan) versetzt und 1 h nachgerührt. Dann versetzt man die Reaktionsmischung mit einer Lösung von 11.8 g (100 mmol) Oxalsäuredimethylester in 100 ml Diethylether, rührt 30 min. bei -78°C nach, lässt die Reaktionsmischung auf 0 °C erwärmen und versetzt mit 200 ml 1 N Ammoniumchlorid-Lösung. Nach 3 h Rühren bei Raumtemperatur trennt man die organische Phase ab, trocknet über Magnesiumsulfat und entfernt dann den Diethylether im Vakuum. Das so erhaltene Öl wird in 200 ml THF gelöst, mit 20 ml 1 N HCl versetzt und 1 h unter Rückfluss erhitzt. Nach Erkalten gießt man die Reaktionsmischung auf ein Gemisch aus 500 g Eis, 300 ml Wasser und 13 ml konz. Ammoniak-Lösung, saugt vom ausgefallenen Feststoff ab, wäscht diesen dreimal mit Wasser und trocknet im Vakuum. Ausbeute: 21.5 g (78 mmol), 78 %.

### B) 1,3,6,8-Tetrachlor-2,4,5,7-tetraazaphenanthren-9,10-dion

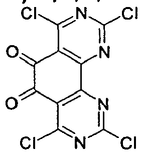

Eine Suspension von 13.8 g (50 mmol) 4H,5H,-2,4,5,7-Tetraazaphenanthren-1,3,6,8,9,10-hexa-on in 90 ml Phosphorylchlorid wird tropfenweise mit 50 ml N,N-Dimethylanilin versetzt. Die Reaktionsmischung wird 20 h unter Rückfluss erhitzt, dann werden bei 50 °C alle flüchtigen Komponenten im Vakuum abgezogen. Der braune Rückstand wird mit 300 ml Wasser versetzt und 1 h gerührt. Man saugt vom braunen Feststoff ab, wäscht diesen dreimal mit je 50 ml Wasser und trocknet dann im Vakuum. Das Rohprodukt wird aus Acetonitril umkristallisiert. Ausbeute: 9.8 g (28 mmol), 56 %.

### C) 1,3,6,8-Tetracyano-2,4,5,7-tetraazaphenanthren-9,10-dion

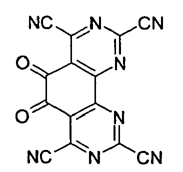

Eine Suspension von 8.75 g (25 mmol) 1,3,6,8-Tetrachlor-2,4,5,7-tetraazaphenanthren-9,10-dion und 9,1 g (140 mmol) Kaliumcyanid in 200 ml wasserfreiem Acetonitril wird 100 h bei Raumtemperatur gerührt. Man gießt die Reaktionsmischung in ein gut gerührtes Gemisch aus 500 g Eis und 300 ml Wasser, saugt vom entstandenen Niederschlag ab, wäscht diesen dreimal mit 50 ml Wasser und trocknet im Vakuum. Das Rohprodukt wird in 2000 ml Acetonitril in der Wärme gelöst und über Alox, sauer, Aktivitätsstufe 1 (300 g) eluiert. Ausbeute: 5.6 g (18 mmol), 72 %.

### D) 2,5,7,9,10,12-Hexacyano-1,3,4,6,8,11-hexaazatriphenylen

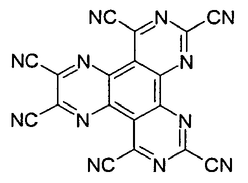

5.3 g (17 mmol) 1,3,6,8-Tetracyano-2,4,5,7-tetraazaphenanthren-9,10-dion und 2.1 g (19 mmol) 2,3-Diaminomaleodinitril [1187-42-4] werden in 250 ml Ethanol und 1 ml Eisessig für 3 h unter Rückfluss erhitzt. Man engt die warme Reaktionsmischung in Vakuum bis zur Trübung ein, lässt über Nacht bei 0 °C stehen, saugt vom auskristallisierten Feststoff ab, löst diesen in 1000 ml Acetonitril in der Wärme und eluiert über Alox, sauer, Aktivitätsstufe 1 (100 g). Nach Entfernen des Acetonitrils im Vakuum wird der Rückstand zweimal fraktioniert sublimiert (p ca. 10⁻⁵ mbar, T = 380 °C). Ausbeute: 4.2 g (11 mmol), 65 %; Reinheit: 99.9 % n. HPLC.

### Beispiel 3: 2,4,6,8,10,12-Hexaphenyl-1,3,5,7,9,11-hexaazatriphenylen

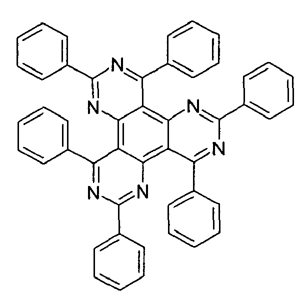

Eine Mischung aus 44.1 g (100 mmol) 2,4,6,8,10,12-Hexachlor1,3,5,7,9,11-hexaazatriphenylen, 97.5 g (800 mmol) Phenylboronsäure, 318.48 g (1.5 mol) Trikaliumphosphat, 5.8 g (5 mmol) Tetrakis-triphenylphosphino-palladium(0), 1500 ml Toluol, 200 ml Dioxan und 1000 ml Wasser wird 12 h unter Rückfluss erhitzt. Nach Erkalten trennt man die organische Phase ab, wäscht diese zweimal mit je 1000 ml Wasser und trocknet über Magnesiumsulfat. Die organische Phase wird im Vakuum bei 70 °C auf ein Volumen von ca. 250 ml eingeengt und dann sukzessive mit 800 ml Ethanol versetzt. Nach Erkalten saugt man vom ausgefallenen Feststoff ab, wäscht diesen dreimal mit Ethanol und trocknet im Vakuum. Nach fünfmaliger Umkristallisation aus DMF wird das Produkt fraktioniert sublimiert (p ca. 10⁻⁶ mbar, T = 360 °C). Ausbeute: 31.8 g (46 mmol), 46 %; Reinheit: 99.9 % n. HPLC.

Analog werden folgende Verbindungen durch Einsatz der entsprechenden Boronsäuren erhalten:

| Bsp. | Boronsäure | Produkt | Ausbeute |
|---|---|---|---|
| 4 | 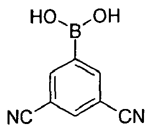 | 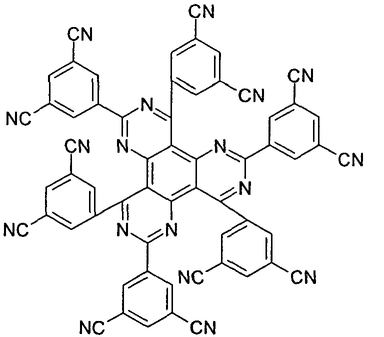 | 33 % |
| | [863868-34-2] | | |
| 5 | 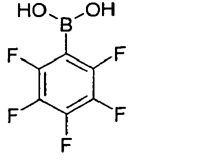 | 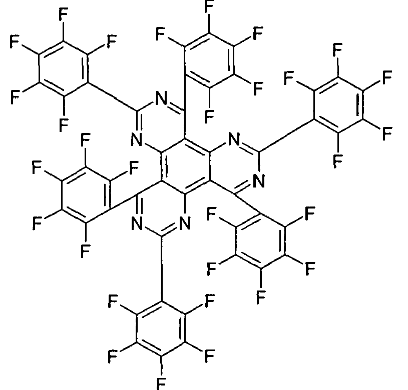 | 21 % |
| | [1582-24-7] | | |
| 6 | 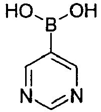 | 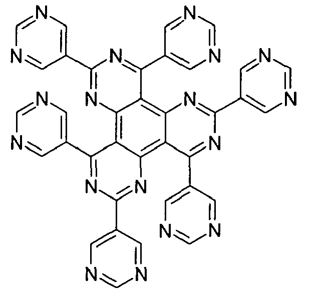 | 38 % |
| | [109299-78-7] | | |
| 7 | 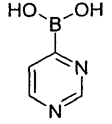 | 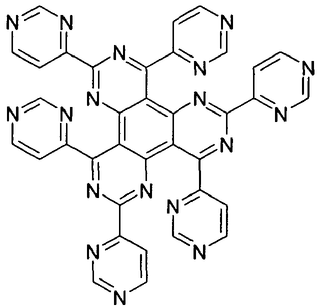 | 30 % |
| | [852362-24-4] | | |

### Beispiel 8: 2,4,6,8,10,12-Hexakis(diphenylamino)-1,3,5,7,9,11-hexaazatriphenylen

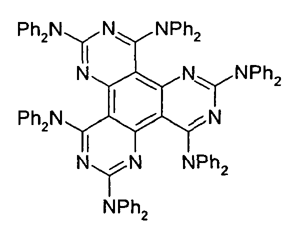

Eine Mischung aus 44.1 g (100 mmol) 2,4,6,8,10,12-Hexachlor1,3,5,7,9,11-hexaazatriphenylen und 135.4 g (800 mmol) Diphenylamin in 500 ml DMF wird portionsweise mit 26.0 g (650 mmol) Natriumhydrid (60 Gew.-%ige Dipersion in Mineralöl) versetzt und nach Abklingen der Gasentwicklung 8 h bei 60 °C gerührt. Nach Erkalten trennt gießt man die Reaktionsmischung vorsichtig auf ein Gemisch aus 1 kg Eis und 500 ml Wasser, extrahiert dann dreimal mit 500 ml Dichlormethan, wäscht die vereinigten Extrakte fünfmal mit je 500 ml Wasser und trocknet die organische Phase über Magnesiumsulfat. Die organische Phase wird über Alox, neutral, Aktivitätsstufe 1 filtriert. Nach Entfernen des Lösungsmittels wird der Rückstand fünfmal aus Dioxan umkristallisiert. Ausbeute: 44.5 g (36 mmol), 36 %; Reinheit: 99.9 % n. HPLC.

### Beispiel 9: Herstellung und Charakterisierung von organischen Elektrolumineszenzvorrichtungen

Die Herstellung von erfindungsgemäßen OLEDs erfolgt nach einem allgemeinen Verfahren gemäß WO 2004/058911, das auf die hier beschriebenen Gegebenheiten (Schichtdickenvariation, verwendete Materialien) angepasst wird.

In den folgenden Beispielen 10 bis 16 werden die Ergebnisse verschiedener OLEDs vorgestellt. Glasplättchen, die mit strukturiertem ITO (Indium Zinn Oxid) beschichtet sind, bilden die Substrate der OLEDs. Zur verbesserten Prozessierung wird 20 nm PEDOT (aus Wasser aufgeschleudert; bezogen von H. C. Starck, Goslar, Deutschland; Poly(3,4-ethylendioxy-2,5-thiophen)) auf das Substrat aufgebracht. Die OLEDs bestehen aus folgender Schichtenfolge: Substrat / PEDOT 20 nm / Lochinjektionsschicht (HIL) 5 nm / Lochtransportschicht (HTL-1) 20 nm / Lochtransportschicht (HTL-2) 20 nm / Emissionschicht (EML) 30 nm / Elektronentransportschicht (ETL) 20 nm und abschließend eine Kathode.

Die Materialien bis auf PEDOT werden in einer Vakuumkammer thermisch aufgedampft. Dabei besteht die Emissionsschicht immer aus einem Matrixmaterial (Host) und einem Dotierstoff (Dotand), der durch Coverdampfung dem Host beigemischt wird. In den unten aufgeführten Beispielen 10 bis 16 wird als Matrixmaterial die Verbindung H1, in den Beispielen 17 bis 19 die Verbindung H2 verwendet, welche jeweils mit 10 % D1 bzw. dem phosphoreszierenden Dotanden D2 dotiert ist. Diese OLEDs zeigen grüne Emission. Als Lochtransportmaterial in der HTL-1 wird die Verbindung HTM-1 verwendet. Als Lochtransportmaterial in der HTL-2 wird NPB verwendet. Die Kathode wird in den Beispielen 10 bis 16 durch eine 1 nm dicke LiF-Schicht und eine darauf abgeschiedene 100 nm dicke Al-Schicht und in den Beipielen 17 bis 19 durch 100 nm dicke Al-Schicht gebildet. Tabelle 1 zeigt die chemischen Strukturen der zum Aufbau der OLEDs verwendeten Materialien.

Diese OLEDs werden standardmäßig charakterisiert; hierfür werden die Elektrolumineszenzspektren, die Effizienz (gemessen in cd/A) in Abhängigkeit der Helligkeit, berechnet aus Strom-Spannungs-Helligkeit-Kennlinien (IUL-Kennlinien), und die Lebensdauer bestimmt. Als Lebensdauer wird die Zeit definiert, nach der die Anfangshelligkeit von 25000 cd/m² bzw. 8000 cd/m² auf die Hälfte gesunken ist. Die Einsatzspannung ist definiert als diejenige Spannung, bei der die OLED eine Helligkeit von 1 cd/m² erreicht.

In Tabelle 2 sind die Ergebnisse einiger OLEDs zusammengefasst. Als Lochinjektionsmaterial werden in der Lochinjektionsschicht (HIL) erfindungsgemäß HIM-1 (2,4,6,8,10,12-Hexacyano-1,3,5,7,9,11-hexaazatriphenylen, aus Beispiel 1), HIM-2 (2,5,7,9,10,12-Hexacyano-1,3,4,6,8,11-hexaazatriphenylen) oder HIM-3 (gemäß dem Stand der Technik) verwendet. Verglichen mit dem Stand der Technik zeichnen sich OLEDs, die HIM-1 oder HIM-2 in der Lochinjektionsschicht enthalten, durch eine verbesserte Effizienz, insbesondere eine verbesserte Leistungseffizienz, und Lebensdauer gegenüber HIM-3 gemäß dem Stand der Technik aus.

Als Elektronentransportmaterial wird in der Elektronentransportschicht (ETL) entweder AlQ₃ gemäß dem Stand der Technik oder eine coverdampfte Kombination von ETM-3 (50%) und ETM-4 (50%) oder erfindungsgemäß 2,4,6,8,10,12-Hexaphenyl-1,3,5,7,9,11-hexaazatriphenylen (ETM-1, gemäß Beispiel 3) oder 2,4,6,8,10,12-Hexa(5-pyrimidinyl)-1,3,5,7,9,11-hexaazatriphenylen (ETM-2, gemäß Beispiel 6) eingesetzt.

**Tabelle 1**

| | |
|---|---|
| 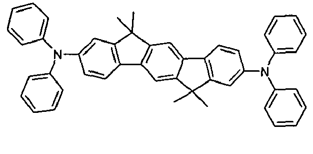 | 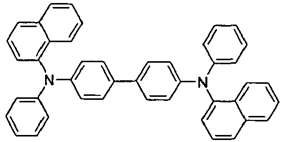 |
| HTM-1 | NPB |
| 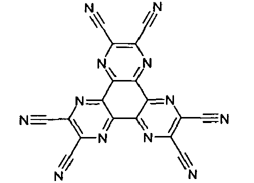 | 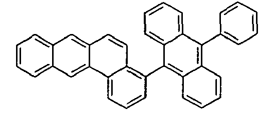 |
| HIM-3 (Stand der Technik) | H1 |
| 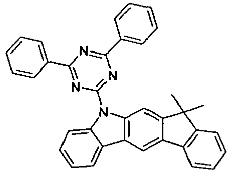 | 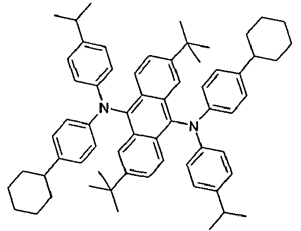 |
| H2 | D1 |
| 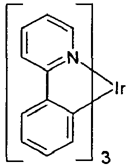 | 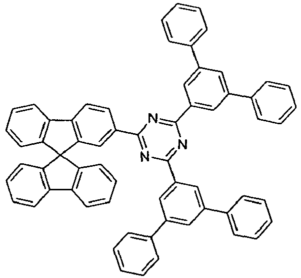 |
| D2 | ETM-3 |
| 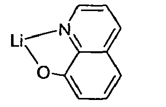 | 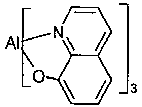 |
| ETM-4 | AlQ₃ (Stand der Technik) |

**Tabelle 2**

| Bsp. | HIL | ETL | Einsatzspannung | Spannung für 1000 cd/m² | Effizienz bei 1000 cd/m² | CIE x/y bei 1000 cd/m² | Lebensdauer für 25000 cd/m² |
|---|---|---|---|---|---|---|---|
| 10 | HIM-1 | AlQ₃ | 2.7 V | 4.8 V | 18.9 | 0.34/0.63 | 410 h |
| | | | | | cd/A | | |
| 11 | HIM-2 | AlQ₃ | 2.7 V | 4.8 V | 18.5 | 0.34/0.63 | 400 h |
| | | | | | cd/A | | |
| 12 | HIM-3 | AlQ₃ | 2.8 V | 5.0 V | 17.1 | 0.34/0.62 | 355 h |
| (Vergl.) | | | | | cd/A | | |
| 13 | HIM-1 | ETM-1 | 2.7 V | 5.0 V | 19.0 | 0.33/0.64 | 465 h |
| | | | | | cd/A | | |
| 14 | HIM-1 | ETM-2 | 2.7 V | 4.8 V | 21.5 | 0.33/0.63 | 450 h |
| | | | | | cd/A | | |
| 15 | HIM-2 | ETM-1 | 2.8 V | 5.0 V | 18.7 | 0.34/0.62 | 400 h |
| | | | | | cd/A | | |
| 16 | HIM-2 | ETM-2 | 2.7 V | 4.7 V | 20.1 | 0.34/0.63 | 435 h |
| | | | | | cd/A | | |
| 17 | HIM-3 | ETM-3 | 2.9 V | 4.5 V | 36.7 | 0.33/0.63 | 430 h |
| (Vergl.) | | ETM-4 | | | | | 8000 cd/m² |
| 18 | HIM-1 | ETM-3 | 2.6 V | 4.0 V | 43.3 | 0.33/0.63 | 500 h |
| | | ETM-4 | | | | | 8000 cd/m² |
| 19 | HIM-2 | ETM-3 | 2.4 V | 3.8 V | 46.0 | 0.33/0.63/ | 540 h |
| | | ETM-4 | | | | | 8000 cd/m² |

## Patentansprüche

1. Verbindung der Formel (2)-(14), 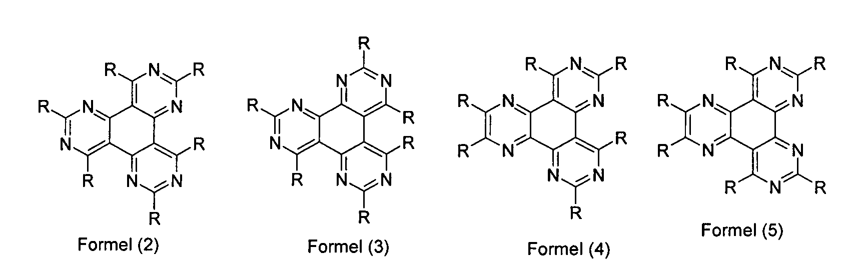 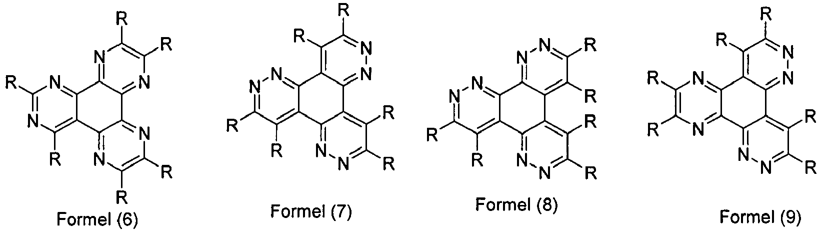 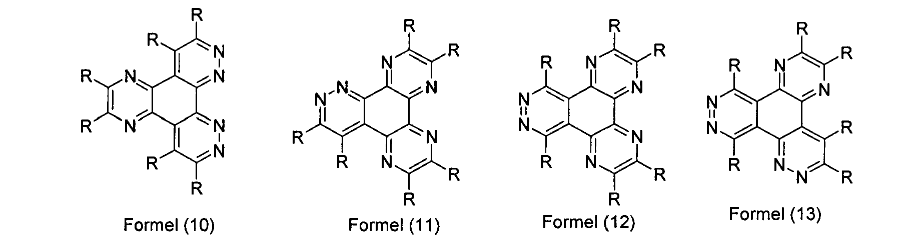 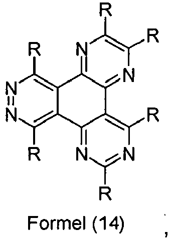 wobei für die verwendeten Symbole gilt:
R ist bei jedem Auftreten gleich oder verschieden H, F, C(=O)Ar, P(=O)(Ar)₂, CN, eine geradkettige Alkylgruppe mit 1 bis 10 C-Atomen oder eine verzweigte oder cyclische Alkylgruppe mit 3 bis 10 C-Atomen, die jeweils mit einem oder mehreren Resten R¹ substituiert sein kann, wobei ein oder mehrere H-Atome durch F oder CN ersetzt sein können, oder ein aromatisches oder heteroaromatisches Ringsystem mit 5 bis 40 aromatischen Ring-atomen, das jeweils durch einen oder mehrere Reste R¹ substituiert sein kann;
R¹ ist bei jedem Auftreten gleich oder verschieden H, D, F, Cl, Br, I, CHO, N(R²)₂, N(Ar)₂, C(=O)Ar, P(=O)(Ar)₂, S(=O)Ar, S(=O)₂Ar, CR²=CR²Ar, CN, NO₂, Si(R²)₃, B(OR²)₂, B(R²)₂, B(N(R²)₂)₂, OSO₂R², eine geradkettige Alkyl-, Alkoxy- oder Thioalkoxygruppe mit 1 bis 40 C-Atomen oder eine geradkettige Alkenyl- oder Alkinyl-gruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R² substituiert sein kann, wobei eine oder mehrere nicht benachbarte CH₂-Gruppen durch R²C=CR², C≡C, Si(R²)₂, Ge(R²)₂, Sn(R²)₂, C=O, C=S, C=Se, C=NR², P(=O)(R²), SO, SO₂, NR², O, S oder CONR² ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R² substituiert sein kann, oder eine Kombination dieser Systeme;
Ar ist bei jedem Auftreten gleich oder verschieden ein aromatisches oder heteroaromatisches Ringsystem mit 5 bis 30 aromatischen Ringatomen, das mit einem oder mehreren nicht-aromatischen Resten R¹ substituiert sein kann; dabei können auch zwei Reste Ar, welche an dasselbe Stickstoff- oder Phosphoratom binden, durch eine Einfachbindung oder eine Brücke, ausgewählt aus B(R²), C(R²)₂, Si(R²)₂, C=O, C=NR², C=C(R²)₂, O, S, S=O, SO₂, N(R²), P(R²) und P(=O)R², miteinander verknüpft sein;
R² ist bei jedem Auftreten gleich oder verschieden H, D oder ein aliphatischer, aromatischer und/oder heteroaromatischer Kohlenwasserstoffrest mit 1 bis 20 C-Atomen, in dem auch H-Atome durch F ersetzt sein können; dabei können zwei oder mehrere benachbarte Substituenten R² auch miteinander ein mono- oder polycyclisches, aliphatisches oder aromatisches Ringsystem bilden.

2. Verbindung nach Anspruch 1, **dadurch gekennzeichnet, dass** der heteroaromatische Grundkörper C₃ₕ- oder C₂ᵥ-Symmetrie aufweist.

3. Verbindung nach Anspruch 1 oder 2, **dadurch gekennzeichnet, dass** entweder alle Reste R gleich gewählt sind oder dass mindestens zwei Reste R unterschiedlich voneinander sind.

4. Verfahren zur Herstellung einer Verbindung nach einem oder mehreren der Ansprüche 1 bis 3, charakterisiert durch die folgenden Reaktionsschritte:
a) Synthese des entsprechenden halogenierten Grundkörpers; und
b) Umsetzung des Halogens zum gewünschten Substituenten.

5. Oligomer, Polymer oder Dendrimer, enthaltend ein oder mehrere Verbindungen nach einem oder mehreren der Ansprüche 1 bis 3, wobei ein oder mehrere Reste R Bindungen der Verbindung zum Polymer, Oligomer oder Dendrimer darstellen.

6. Verwendung einer Verbindung nach einem oder mehreren Ansprüche 1 bis 3 oder 5 in einer elektronischen Vorrichtung.

7. Elektronische Vorrichtung, insbesondere ausgewählt aus der Gruppe bestehend aus organischen Elektrolumineszenzvorrichtungen, organischen integrierten Schaltungen, organischen Feld-Effekt-Transistoren, organischen Dünnfilmtransistoren, organischen lichtemittierenden Transistoren, organischen Solarzellen, farbstoffsensibilisierten Solarzellen, organischen optischen Detektoren, organischen Photorezeptoren, organischen Feld-Quench-Devices, lichtemittierenden elektrochemischen Zellen, organischen Laserdioden und "organic plasmon emitting devices", enthaltend mindestens eine Verbindung nach einem oder mehreren der Ansprüche 1 bis 3 oder 5.

8. Organische Elektrolumineszenzvorrichtung nach Anspruch 7, **dadurch gekennzeichnet, dass** die Verbindung nach einem oder mehreren der Ansprüche 1 bis 3 oder 5 in einer Lochinjektions- bzw. Lochtransportschicht verwendet wird.

9. Organische Elektrolumineszenzvorrichtung nach Anspruch 8, **dadurch gekennzeichnet, dass** sie einen der folgenden Aufbauten a) oder b) aufweist:
a) Anode - Lochinjektionsschicht enthaltend mindestens eine Verbindung nach einem oder mehreren der Ansprüche 1 bis 3 oder 5-Lochtransportschicht - emittierende Schicht - Kathode; oder
b) Anode - Lochinjektionsschicht - Lochtransportschicht, enthaltend mindestens eine Verbindung nach einem oder mehreren der Ansprüche 1 bis 3 oder 5 - Lochtransportschicht - emittierende Schicht-Kathode,
wobei die Elektrolumineszenzvorrichtung außer den genannten Schichten noch weitere Schichten aufweisen kann.

10. Organische Elektrolumineszenzvorrichtung nach einem oder mehreren der Ansprüche 7 bis 9, **dadurch gekennzeichnet, dass** die Verbindung nach einem oder mehreren der Ansprüche 1 bis 3 oder 5 als Elektronentransportmaterial bzw. als Lochblockiermaterial in einer Elektronentransportschicht bzw. in einer Lochblockierschicht eingesetzt wird und/oder dass die Verbindung nach einem oder mehreren der Ansprüche 1 bis 3 oder 5 als Ladungserzeugungsmaterial in einer Ladungserzeugungsschicht eingesetzt wird und/oder dass die Verbindung nach einem oder mehreren der Ansprüche 1 bis 3 oder 5 als Matrixmaterial für eine emittierende Verbindung, insbesondere für eine phosphoreszierende Verbindung, eingesetzt wird.

## Claims

1. Compound of the formula (2)-(14), 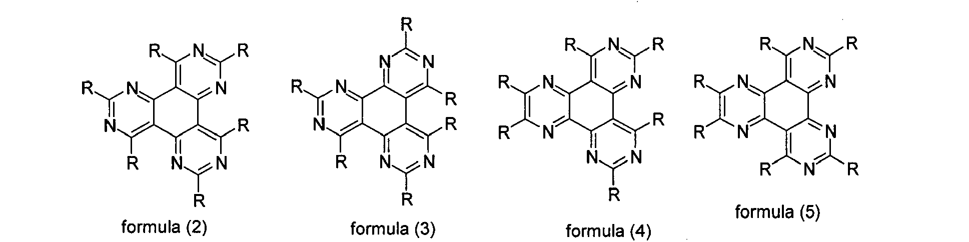 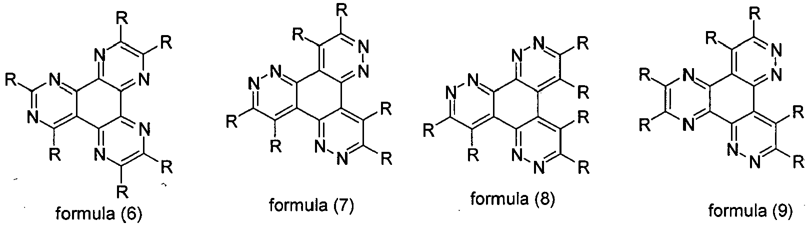 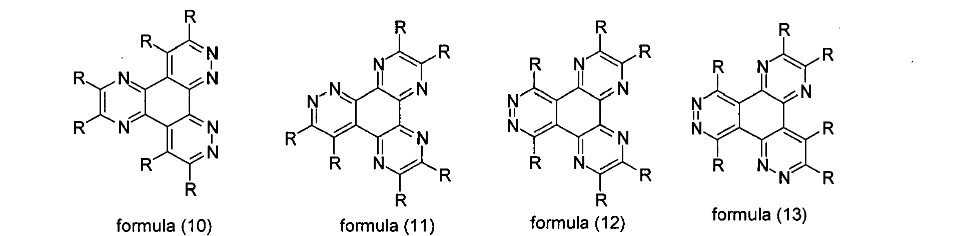 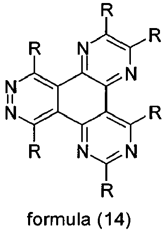 where the following applies to the symbols used:
R is on each occurrence, identically or differently, H, F, C(=O)Ar, P(=O)(Ar)₂, CN, a straight-chain alkyl group having 1 to 10 C atoms or a branched or cyclic alkyl group having 3 to 10 C atoms, each of which may be substituted by one or more radicals R¹, where one or more H atoms may be replaced by F or CN, or an aromatic or heteroaromatic ring system having 5 to 40 aromatic ring atoms, which may in each case be substituted by one or more radicals R¹;
R¹ is on each occurrence, identically or differently, H, D, F, Cl, Br, I, CHO, N(R²)₂, N(Ar)₂, C(=O)Ar, P(=O)(Ar)₂ S(=O)Ar, S(=O)₂Ar, CR²=CR²Ar, CN, NO₂, Si(R²)₃, B(OR²)₂, B(R²)₂, B(N(R²)₂)₂, OSO₂R², a straight-chain alkyl, alkoxy or thioalkoxy group having 1 to 40 C atoms or a straight-chain alkenyl or alkynyl group having 2 to 40 C atoms or a branched or cyclic alkyl, alkenyl, alkynyl, alkoxy or thioalkoxy group having 3 to 40 C atoms, each of which may be substituted by one or more radicals R², where one or more non-adjacent CH₂ groups may be replaced by R²C=CR², C=C, Si(R²)₂, Ge(R²)₂, Sn(R²)₂, C=O, C=S, C=Sₑ, C=NR², P(=O)(R²), SO SO_{2,} NR², O, S or CONR² and where one or more H atoms may be replaced by D, F, Cl, Br, I, CN or NO₂, or an aromatic or heteroaromatic ring system having 5 to 60 aromatic ring atoms, which may in each case be substituted by one or more radicals R², or an aryloxy or heteroaryloxy group having 5 to 60 aromatic ring atoms, which may be substituted by one or more radicals R², or a combination of these systems;
Ar is on each occurrence, identically or differently, an aromatic or heteroaromatic ring system having 5 to 30 aromatic ring atoms, which may be substituted by one or more non-aromatic radicals R¹; two radicals Ar which are bonded to the same nitrogen or phosphorus atom may also be linked to one another here by a single bond or a bridge selected from B(R²), C(R²)₂, Si(R²)₂, C=O, C=NR², C=C(R²)₂, O, S, S=O, SO₂, N(R²), P(R²) and P(=O)R²;
R² is on each occurrence, identically or differently, H, D or an aliphatic, aromatic and/or heteroaromatic hydrocarbon radical having 1 to 20 C atoms, in which, in addition, H atoms may be replaced by F; two or more adjacent substituents R² here may also form a mono- or polycyclic, aliphatic or aromatic ring system with one another.

2. Compound according to Claim 1, **characterised in that** the heteroaromatic skeleton has C₃ₕ or C₂ᵥ symmetry.

3. Compound according to Claim 1 or 2, **characterised in that** either all radicals R are selected identically or **in that** at least two radicals R are different from one another.

4. Process for the preparation of a compound according to one or more of Claims 1 to 3, **characterised by** the following reaction steps:
a) synthesis of the corresponding halogenated skeleton; and
b) conversion of the halogen into the desired substituent.

5. Oligomer, polymer or dendrimer containing one or more compounds according to one or more of Claims 1 to 3, where one or more radicals R represent bonds from the compound to the polymer, oligomer or dendrimer.

6. Use of a compound according to one or more of Claims 1 to 3 or 5 in an electronic device.

7. Electronic device, in particular selected from the group consisting of organic electroluminescent devices, organic integrated circuits, organic field-effect transistors, organic thin-film transistors, organic light-emitting transistors, organic solar cells, dye-sensitised solar cells, organic optical detectors, organic photoreceptors, organic field-quench devices, light-emitting electrochemical cells, organic laser diodes and "organic plasmon emitting devices", comprising at least one compound according to one or more of Claims 1 to 3 or 5.

8. Organic electroluminescent device according to Claim 7, **characterised in that** the compound according to one or more of Claims 1 to 3 or 5 is used in a hole-injection or hole-transport layer.

9. Organic electroluminescent device according to Claim 8, **characterised in that** it has one of the following structures a) or b):
a) anode - hole-injection layer comprising at least one compound according to one or more of Claims 1 to 3 or 5 - hole-transport layer - emitting layer - cathode; or
b) anode - hole-injection layer - hole-transport layer comprising at least one compound according to one or more of Claims 1 to 3 or 5-hole-transport layer - emitting layer - cathode;
where the electroluminescent device may also comprise further layers in addition to the said layers.

10. Organic electroluminescent device according to one or more of Claims 7 to 9, **characterised in that** the compound according to one or more of Claims 1 to 3 or 5 is employed as electron-transport material or as hole-blocking material in an electron-transport layer or in a hole-blocking layer and/or **in that** the compound according to one or more of Claims 1 to 3 or 5 is employed as charge-generation material in a charge-generation layer and/or **in that** the compound according to one or more of Claims 1 to 3 or 5 is employed as matrix material for an emitting compound, in particular for a phosphorescent compound.

## Revendications

1. Composé des formules (2)-(14) : 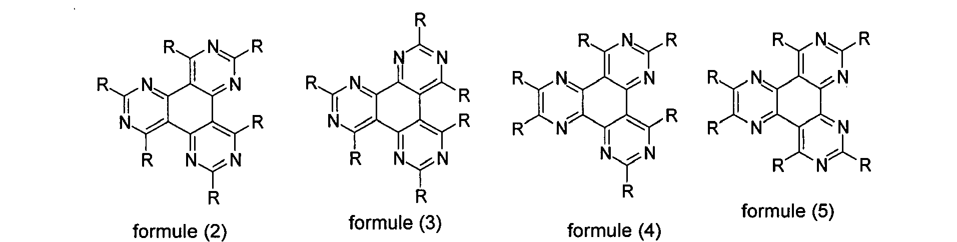 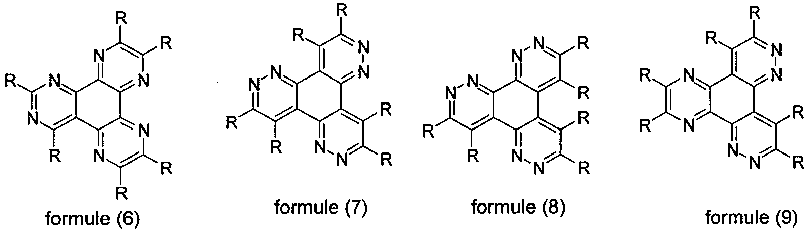 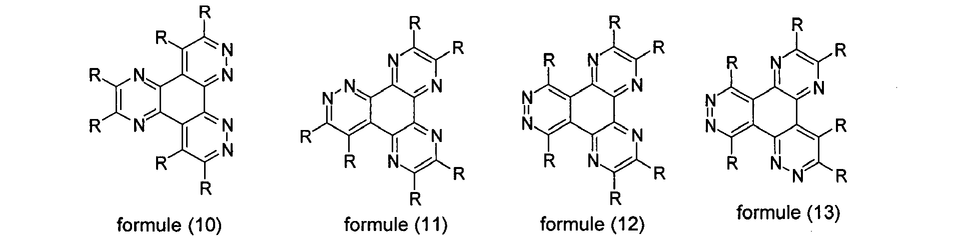 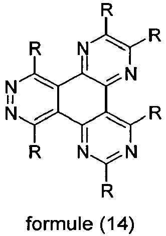 dans lesquelles ce qui suit s'applique aux symboles utilisés :
R est, pour chaque occurrence, de manière identique ou différente, H, F, C(=O)Ar, P(=O)(Ar)₂, CN, un groupe alkyle en chaîne droite comportant 1 à 10 atome(s) de C ou un groupe alkyle ramifié ou cyclique comportant 3 à 10 atomes de C, dont chacun peut être substitué par un radical ou plusieurs radicaux R¹, où un ou plusieurs atome(s) de H peut/peuvent être remplacé(s) par F ou CN, ou un système de cycle aromatique ou hétéroaromatique comportant 5 à 40 atomes de cycle aromatique, lequel peut dans chaque cas être substitué par un radical ou plusieurs radicaux R¹ ;
R¹ est, pour chaque occurrence, de manière identique ou différente, H, D, F, Cl, Br, I, CHO, N(R²)₂, N(Ar)₂, C(=O)Ar, P(=O)(Ar)₂, S(=O)Ar, S(=O)₂Ar, CR²=CR²Ar, CN, NO₂, Si(R²)₃, B(OR²)₂, B(R²)₂, B(N(R²)₂)₂, OSO₂R², un groupe alkyle, alcoxy ou thioalcoxy en chaîne droite comportant 1 à 40 atome(s) de C ou un groupe alkényle ou alkynyle en chaîne droite comportant 2 à 40 atomes de C ou un groupe alkyle, alkényle, alkynyle, alcoxy ou thioalcoxy ramifié ou cyclique comportant 3 à 40 atomes de C, dont chacun peut être substitué par un radical ou plusieurs radicaux R², où un ou plusieurs groupe(s) CH₂ non adjacents peut/peuvent être remplacé(s) par R²C=CR², C=C, Si(R²)₂, Ge(R²)₂, Sn(R²)₂, C=O, C=S, C=Se, C=NR², P(=O)(R²), SO, SO₂, NR², O, S ou CONR² et où un ou plusieurs atome(s) de H peut/peuvent être remplacé(s) par D, F, Cl, Br, I, CN ou NO₂, ou un système de cycle aromatique ou hétéroaromatique comportant 5 à 60 atomes de cycle aromatique, lequel peut dans chaque cas être substitué par un radical ou plusieurs radicaux R², ou un groupe aryloxy ou hétéroaryloxy comportant 5 à 60 atomes de cycle aromatique, lequel peut être substitué par un radical ou plusieurs radicaux R², ou une combinaison de ces systèmes ;
Ar est, pour chaque occurrence, de manière identique ou différente, un système de cycle aromatique ou hétéroaromatique comportant 5 à 30 atomes de cycle aromatique, lequel peut être substitué par un radical ou plusieurs radicaux non aromatique(s) R¹ ; deux radicaux Ar qui sont liés au même atome d'azote ou de phosphore peuvent également être reliés l'un à l'autre ici par une liaison simple ou par un pont choisi parmi B(R²), C(R²)₂, Si(R²)₂, C=O, C=NR², C=C(R²)₂, O, S, S=O, SO₂, N(R²), P(R²) et P(=O)R² ;
R² est, pour chaque occurrence, de manière identique ou différente, H, D ou un radical hydrocarbone aliphatique, aromatique et/ou hétéroaromatique comportant 1 à 20 atome(s) de C, où, en outre, des atomes de H peuvent être remplacés par F ; deux substituants adjacents R² ou plus peuvent ici également former un système de cycle aliphatique ou aromatique monocyclique ou polycyclique l'un avec l'autre ou les uns avec les autres.

2. Composé selon la revendication 1, **caractérisé en ce que** le squelette hétéroaromatique présente une symétrie C₃ₕ ou C₂ᵥ.

3. Composé selon la revendication 1 ou 2, caractérisé soit en ce que tous les radicaux R sont choisis de manière identique, soit en ce qu'au moins deux radicaux R sont différents l'un de l'autre ou les uns des autres.

4. Procédé pour la préparation d'un composé selon une ou plusieurs des revendications 1 à 3, **caractérisé par** les étapes de réaction qui suivent :
a) synthèse du squelette halogéné correspondant ; et
b) conversion de l'halogène selon le substituant souhaité.

5. Oligomère, polymère ou dendrimère contenant un ou plusieurs composé(s) selon une ou plusieurs des revendications 1 à 3, où un radical ou plusieurs radicaux R représente/représentent des liaisons depuis le composé sur le polymère, l'oligomère ou le dendrimère.

6. Utilisation d'un composé selon une ou plusieurs des revendications 1 à 3 ou 5 dans un dispositif électronique.

7. Dispositif électronique, en particulier sélectionné parmi le groupe constitué par les dispositifs électroluminescents organiques, les circuits intégrés organiques, les transistors à effet de champ organiques, les transistors à film mince organiques, les transistors à émission de lumière organiques, les cellules solaires organiques, les cellules solaires sensibilisées par colorant, les détecteurs optiques organiques, les photorécepteurs organiques, les dispositifs à extinction de champ organiques, les cellules électrochimiques à émission de lumière, les diodes laser organiques et les "dispositifs à émission de plasmons organiques", comprenant au moins un composé selon une ou plusieurs des revendications 1 à 3 ou 5.

8. Dispositif électroluminescent organique selon la revendication 7, **caractérisé en ce que** le composé selon une ou plusieurs des revendications 1 à 3 ou 5 est utilisé dans une couche d'injection de trous ou de transport de trous.

9. Dispositif électroluminescent organique selon la revendication 8, **caractérisé en ce qu'**il présente l'une des structures qui suivent a) ou b) :
a) une anode - une couche d'injection de trous comprenant au moins un composé selon une ou plusieurs des revendications 1 à 3 ou 5 - une couche de transport de trous - une couche d'émission - une cathode ; ou
b) une anode - une couche d'injection de trous - une couche de transport de trous comprenant au moins un composé selon une ou plusieurs des revendications 1 à 3 ou 5 - une couche de transport de trous - une couche d'émission - une cathode ;
où le dispositif électroluminescent peut également comprendre d'autres couches en plus desdites couches.

10. Dispositif électroluminescent organique selon une ou plusieurs des revendications 7 à 9, **caractérisé en ce que** le composé selon une ou plusieurs des revendications 1 à 3 ou 5 est utilisé en tant que matériau de transport d'électrons ou en tant que matériau de blocage de trous dans une couche de transport d'électrons ou dans une couche de blocage de trous et/ou **en ce que** le composé selon une ou plusieurs des revendications 1 à 3 ou 5 est utilisé en tant que matériau de génération de charges dans une couche de génération de charge et/ou **en ce que** le composé selon une ou plusieurs des revendications 1 à 3 ou 5 est utilisé en tant que matériau de matrice pour un composé d'émission, en particulier pour un composé phosphorescent.
